# EUROPEAN PATENT APPLICATION

(11) **EP 4 516 909 A1**
(43) Date of publication of application: **05.03.2025**
(21) Application number: 23795589.3
(22) Date of filing: 28.04.2023
(51) Int. Cl.: C12N 15/62, C12N 5/10, C07K 19/00, C07K 16/28, A61K 35/17, A61K 39/395, A61P 35/00, A61P 35/02

(54) **CD19-TARGETING CHIMERIC ANTIGEN RECEPTOR AND USE THEREOF**

(30) Priority: 29.04.2022 CN 202210468137
(71) Applicant: Shanghai Simnova Biotechnology Co., Ltd., Shanghai 201321 (CN)
(72) Inventor: JIANG, Fuwei, Shanghai 201318 (CN); WANG, Chao, Shanghai 201318 (CN); WANG, Qingyang, Shanghai 201318 (CN); YANG, Cuiqing, Shanghai 201318 (CN); WANG, Pin, Shanghai 201318 (CN); CAO, Zhuoxiao, Shanghai 201318 (CN)
(74) Representative: Richly & Ritschel Patentanwälte PartG mbB
(86) International application number: PCT/CN2023/091417
(87) International publication number: WO 2023/208157

(57) **Abstract**

Provided are a CD19-targeting chimeric antigen receptor and use thereof. Further provided are a CD19-targeting chimeric antigen receptor, a corresponding nucleic acid molecule, a vector, an immune effector cell, a preparation method therefor, a product thereof, a pharmaceutical composition, a therapeutic use, a pharmaceutical use, and a tumor or cancer treatment method.

## Description

### CROSS-REFERENCE TO RELATED APPLICATION

The present application claims priority to the Chinese Patent Application No. 202210468137.4 filed on April 29, 2022, the content of which is incorporated herein by reference in its entirety and for all purposes.

### TECHNICAL FIELD

The present application generally relates to the field of bioengineering and cell therapy, and in particular, to a chimeric antigen receptor targeting CD19 and use thereof.

### BACKGROUND

CD19 belongs to a member of the immunoglobulin superfamily and is a 95-kDa type I transmembrane glycoprotein consisting of a single transmembrane domain, an N-terminal extracellular domain, and a C-terminal intracellular domain. The N-terminal extracellular domain consists of two C2-type Ig-like domain. The C-terminal intracellular domain is highly conserved, and consists of 242 amino acids and 9 tyrosine residues near the C-terminus. CD19 plays an important role in regulating the activation and proliferation process of B lymphocytes, and is widely expressed on the surface of B cell malignancy cells, mainly including chronic lymphocytic leukemia (CLL), acute lymphocytic leukemia (ALL), non-Hodgkin's lymphoma (diffuse large B-cell lymphoma DLBCL, primary mediastinal B-cell lymphoma PMBCL, and follicular lymphoma FL), and the like.

In recent years, cell immunotherapy technology, particularly those based on chimeric antigen receptors, has made breakthrough progress. CD19 has become a hot target for chimeric antigen receptor technology, and CAR-T products specifically targeting CB19 have been approved for marketing by FDA of the United States. However, the existing CD19 CART product uses a murine antibody FMC63, and has immunogenicity, so that an anti-drug antibody (ADA) or a cytotoxic T lymphocyte (CTL) of the heterologous antibody used for the CAR-T is easily generated in a human body, and the CAR-T cells in the patients are rapidly eliminated to cause disease relapse.

In addition, although CAR-modified T cells have anti-tumor activity, there are still limitations in terms of scheduling and clinical aspects. CAR T cells are prepared for individual patients, and thus the preparation is complex in process and expensive. Some patients receive CAR T cell therapy with significant toxic effects (including cytokine release syndrome and neurotoxicity thus requiring treatment in a specialized therapeutic institution). An allogeneic product that is effective and has better safety would overcome these limitations.

### SUMMARY

In a first aspect, the present application provides a nucleic acid molecule encoding a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 9-11, respectively, and the LCDR1-3 have the sequences set forth in SEQ ID NOs: 12-14, respectively; or
(2) the HCDR1-3 have the sequences set forth in SEQ ID NOs: 15-17, respectively, and the LCDR1-3 have the sequences set forth in SEQ ID NOs: 18-20, respectively; or
(3) the HCDR1-3 have the sequences set forth in SEQ ID NOs: 21-23, respectively, and the LCDR1-3 have the sequences set forth in SEQ ID NOs: 24-26, respectively; or
(4) the HCDR1-3 have the sequences set forth in SEQ ID NOs: 27-29, respectively, and the LCDR1-3 have the sequences set forth in SEQ ID NOs: 30-32, respectively; or
(5) the HCDR1-3 have at most 0-3 mutations compared with each corresponding CDR in any one of groups (1) - (4), respectively, and the LCDR1-3 have at most 0-3 mutations compared with each corresponding CDR in any one of groups (1) - (4), respectively.

In a second aspect, the present application provides a chimeric antigen receptor targeting CD19 encoded by the nucleic acid molecule according to the first aspect.

In a third aspect, the present application provides a vector comprising the nucleic acid molecule according to the first aspect.

In a fourth aspect, the present application provides an immune effector cell, wherein the immune effector cell is introduced with the nucleic acid molecule according to the first aspect or is introduced with the vector according to the third aspect or expresses the chimeric antigen receptor according to the second aspect.

In a fifth aspect, the present application provides a method for preparing an immune effector cell, wherein the method comprises the steps of: providing an immune effector cell, and introducing the nucleic acid molecule according to the first aspect or the vector according to the third aspect into the immune effector cell; optionally, the method further comprises culturing the immune effector cell after introducing the nucleic acid molecule or the vector.

In a sixth aspect, the present application provides a product prepared according to the method according to the fifth aspect.

In a seventh aspect, the present application provides a pharmaceutical composition comprising the nucleic acid molecule according to the first aspect or the vector according to the third aspect or the immune effector cell according to the fourth aspect or the product according to the sixth aspect, and a pharmaceutically acceptable carrier.

In an eighth aspect, the present application provides use of the nucleic acid molecule according to the first aspect or the vector according to the third aspect or the immune effector cell according to the fourth aspect or the product according to the sixth aspect or the pharmaceutical composition according to the seventh aspect in the manufacture of a medicament for treating a tumor or cancer, and the tumor or cancer is preferably a CD19-related tumor or cancer, more preferably a B cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

In a ninth aspect, the present application provides a method for treating a cancer or tumor, wherein the method comprises administering to a subject in need an effective amount of the nucleic acid molecule according to the first aspect or the vector according to the third aspect or the immune effector cell according to the fourth aspect or the product according to the sixth aspect or the pharmaceutical composition according to the seventh aspect, and the tumor or cancer is preferably a CD19-related tumor or cancer, more preferably a B cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

### BRIEF DESCRIPTION OF THE DRAWINGS

FIG. 1 shows a schematic diagram of the structure of a chimeric antigen receptor targeting CD19.
FIG. 2 shows the *in vitro* killing effect of different CD19 CAR NKs on Nalm6 cells, wherein three bars of each group show results at effector-to-target ratios (E:T) of 0.5:1, 1:1, and 2:1 from left to right, and NT represents the NK cell without transfected CAR.
FIG. 3 shows the *in vitro* killing effect of different CD19 CAR NKs on Raji cells, wherein three bars of each group show results at effector-to-target ratios (E:T) of 0.5:1, 1:1, and 2:1 from left to right, and NT represents the NK cell without transfected CAR.
FIG. 4 shows the *in vitro* killing effect of different CD19 CAR NKs on Molm13 cells, wherein three bars of each group show results at effector-to-target ratios (E:T) of 0.5:1, 1:1, and 2:1 from left to right, and NT represents the NK cell without transfected CAR.
FIG. 5 shows the tumor imaging results of the Nalm6 xenograft model in animals after receiving different CD19 CAR NK treatments, wherein the NT group corresponds to blank NK in Table 5, the Mab01-CAR1 group corresponds to Mab01-CAR in Table 5, the Mab03-CAR group corresponds to Mab03-CAR3 in Table 5, and the FMC63-CAR group corresponds to FMC63-CAR4 in Table 5.
FIG. 6A shows the expansion of different CD19 CAR NKs, wherein UT represents untreated.
FIG. 6B shows the CAR cell positive rate of different CD19 CAR NKs during expansion culture.
FIGs. 7A and 7B show the killing effect of different CD19 CAR NKs on Raji cells, wherein FIG. 7A shows the results at 0 h of thawing, and FIG. 7B shows the results at 24 h after thawing.
FIGs. 8A and 8B show the tumor imaging results of the Nalm6 xenograft model in animals after receiving different CD19 CAR NK treatments, wherein FIG. 8A shows the results on days 5-26 and FIG. 8B shows the results on days 30-33.
FIG. 8C shows the photon count results of the tumor imaging of the Nalm6 xenograft model in animals after receiving different CD19 CAR NK treatments (on day 19, animals in the negative control group started to die, so the photon count data was only recorded up to day 19).
FIG. 8D shows the body weight results of the animals of the Nalm6 xenograft model after receiving different CD19 CAR NK treatments (on day 19, animals in the negative control group started to die, so the body weight data was only recorded up to day 19).
FIG. 8E shows the survival curves of the animals of the Nalm6 xenograft model after receiving different CD19 CAR NK treatments.
FIG. 9A shows the proportion of CD56⁺CAR19⁺CD3⁻ cells in peripheral blood samples obtained from the animals of the Nalm6 xenograft model receiving different CD19 CAR NK treatments.
FIG. 9B shows the proportion of CD56⁺CAR19⁻CD3⁻ cells in peripheral blood samples obtained from the animals of the Nalm6 xenograft model receiving different CD19 CAR NK treatments.
FIG. 10 shows a summary of efficacy assay of the Nalm6 xenograft model for different CD19 CAR NK treatment groups.

### DETAILED DESCRIPTION

### TERMINOLOGY AND DEFINITIONS

Unless otherwise defined herein, scientific and technical terms used in correlation with the present application shall have the meanings that are commonly understood by those skilled in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

The terms "including", "comprising", and "having" herein are used interchangeably and are intended to indicate the inclusion of a solution, implying that there may be elements other than those listed in the solution. Meanwhile, it should be understood that the descriptions "including", "comprising", and "having" as used herein also provide the solution of "consisting of ...". Illustratively, "a composition, comprising A and B" should be understood as the following technical solution: a composition consisting of A and B, and a composition containing other components in addition to A and B, and all fall within the scope of the aforementioned "a composition".

The term "and/or" as used herein includes the meanings of "and", "or", and "all or any other combination of elements linked by the term".

Although the numerical ranges and parameter approximations are shown in the broad scope of the present application, the numerical values shown in the specific examples are recorded as accurately as possible. However, any numerical values inherently contain certain errors necessarily resulting from the standard deviations found in their respective measurements. In addition, all ranges disclosed herein should be understood to encompass any and all subranges subsumed therein. For example, a recorded range of "1 to 10" should be considered to include any and all subranges between the minimum value of 1 and the maximum value of 10 (including the endpoints); that is, all subranges starting with a minimum value of 1 or greater, such as 1 to 6.1, and all subranges ending with a maximum value of 10 or less, such as 5.5 to 10. Additionally, any reference referred to as "incorporated herein" should be understood as being incorporated in its entirety.

As used herein, "chimeric antigen receptor (CAR)" refers to an artificial immune effector cell surface receptor engineered to be expressed on an immune effector cell and specifically bound to an antigen, which at least comprises (1) an extracellular antigen-binding domain, e.g., a variable heavy or light chain of an antibody, (2) a transmembrane domain that anchors the CAR into the immune effector cell, and (3) an intracellular signaling domain. The CAR is capable of redirecting T cells and other immune effector cells to a selected target, e.g., a cancer cell, in a non-MHC-restricted manner using the extracellular antigen-binding domain. The extracellular domain of a chimeric antigen receptor may also include a signal peptide and/or a hinge region. The intracellular domain of a chimeric antigen receptor may also include a costimulatory domain.

As used herein, the term "signal peptide" in the context of a chimeric antigen receptor refers to a fragment of a protein or polypeptide that is used to direct the protein or polypeptide into the secretory pathway and transfer them to the cell membrane and/or cell surface. A non-limiting example of a signal peptide is the CD8α signal peptide.

As used herein, the term "hinge region" in the context of a chimeric antigen receptor generally refers to any oligopeptide or polypeptide that functions to connect a transmembrane region and an antigen-binding region. Specifically, the hinge region serves to provide greater flexibility and accessibility to the antigen-binding region. The hinge region may be derived in whole or in part from a natural molecule, such as in whole or in part from an extracellular region of CD8, CD4, or CD28, or in whole or in part from an antibody constant region. Alternatively, the hinge region may be a synthetic sequence corresponding to a naturally occurring hinge sequence, or may be a completely synthetic hinge sequence.

As used herein, the term "transmembrane (TM) region" in the context of a chimeric antigen receptor refers to a polypeptide structure that enables expression of the chimeric antigen receptor on the surface of an immune cell (e.g., a lymphocyte, an NK cell, or an NKT cell) and directs the cellular response of the immune cell against a target cell. The transmembrane domain may be natural or synthetic, and may be derived from any membrane-bound protein or transmembrane protein. The transmembrane domain is capable of signaling when the chimeric antigen receptor binds to a target antigen. A non-limiting example of a hinge region is the CD8 transmembrane region.

As used herein, the term "intracellular signaling domain" in the context of a chimeric antigen receptor refers to a protein portion that transduces an effector function signal and directs a cell to perform a specified function. The intracellular signaling domain is responsible for intracellular primary signaling after the antigen-binding domain binds to the antigen, resulting in activation of the immune cell and immune response. In other words, the intracellular signaling domain is responsible for activating at least one of the normal effector functions of the immune cell in which the CAR is expressed. Exemplary intracellular signaling domains include CD3ζ.

As used herein, the term "costimulatory domain" in the context of a chimeric antigen receptor refers to the intracellular signaling domain of a costimulatory molecule. Costimulatory molecules are cell surface molecules other than antigen receptors or Fc receptors that provide the second signal required for effective activation and functioning of T lymphocytes upon binding to the antigen. A non-limiting example of a costimulatory domain is the CD28 costimulatory domain.

The term "immune effector cell" or "effector cell" as used herein refers to a cell involved in an immune response, e.g., promoting an immune effector response. Examples of immune effector cells include T cells, e.g., α/β T cells and γ/δ T cells, B cells, natural killer (NK) cells, natural killer T (NKT) cells, mast cells, and myeloid-derived phagocytes.

The term "specific binding" herein means that an antigen-binding molecule (e.g., an antibody or a ligand of an antigen) specifically binds to an antigen and substantially identical antigens, generally with high affinity, but does not bind to unrelated antigens with high affinity. Affinity is generally reflected in an equilibrium dissociation constant (KD), where a relatively low KD indicates a relatively high affinity. In the case of antibodies, high affinity generally means having a KD of about 10⁻⁶ M or less, about 10⁻⁷ M or less, about 10⁻⁸ M or less, about 1 × 10⁻⁹ M or less, about 1 × 10⁻¹⁰ M or less, 1 × 10⁻¹¹ M or less, or 1 × 10⁻¹² M or less. KD is calculated as follows: KD = Kd/Ka, where Kd represents the dissociation rate and Ka represents the association rate. The equilibrium dissociation constant KD can be measured by methods well known in the art, such as surface plasmon resonance (e.g., Biacore) or equilibrium dialysis. Illustratively, KD can be obtained by the method as described in the examples herein.

The term "antibody" herein is used in its broadest sense and refers to a polypeptide or a combination of polypeptides that comprises sufficient sequence from an immunoglobulin heavy chain variable region and/or sufficient sequence from an immunoglobulin light chain variable region to be capable of specifically binding to an antigen. "Antibody" herein encompasses various forms and various structures as long as they exhibit the desired antigen-binding activity, including intact antibodies and antigen-binding fragments thereof.

The term "antibody" herein includes a typical "four-chain antibody", which is an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is of IgE isoform, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are connected to each other and to the light chains through disulfide bonds to form a Y-shaped structure. The heavy chain constant regions of immunoglobulins differ in their amino acid composition and arrangement, and thus in their antigenicity. Accordingly, "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. The light chains can be divided into κ or λ chains according to the differences in the constant regions. Each of the five classes of Ig may have a κ chain or a λ chain.

"Antigen-binding fragment" and "antibody fragment" herein are used interchangeably and do not have the entire structure of an intact antibody, but comprise only a portion of the intact antibody or a variant of the portion that has the ability to bind to an antigen. "Antigen-binding fragment" or "antibody fragment" herein includes but is not limited to, a Fab, a Fab', a Fab'-SH, a F(ab')₂, an scFv, and a VHH.

The term "scFv" (single-chain variable fragment) herein refers to a single polypeptide chain comprising VL and VH domains, wherein the VL and VH are linked through a linker (see, e.g., Bird et al., Science 242: 423-426 (1988); Huston et al., Proc. Natl. Acad. Sci. USA 85: 5879-5883 (1988); and Pluckthun, The Pharmacology of Monoclonal Antibodies, Vol. 113, Roseburg and Moore Ed., Springer-Verlag, New York, pp 269-315 (1994)). Such scFv molecules may have a general structure: NH2-VL-linker-VH-COOH or NH2-VH-linker-VL-COOH. An appropriate linker in the prior art includes GSTSGSGKPGSGEGSTKG and consists of GGGGS amino acid sequence repeats or a variant thereof. For example, a linker having the amino acid sequence (GGGGS)₄ can be used, and variants thereof can also be used (Holliger et al. (1993), Proc. Natl. Acad. Sci. USA 90: 6444-6448). Other linkers that can be used in the present disclosure are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56; and Roovers et al. (2001), Cancer Immunol*.* In some cases, there may also be disulfide bonds between the VH and VL of the scFv, forming a disulfide-linked Fv (dsFv).

"Antibody" herein may be derived from any animal, including, but not limited to, human and non-human animals which may be selected from primates, mammals, rodents, and vertebrates, such as Camelidae species, *Lama glama*, *Lama guanicoe*, *Vicugna pacos*, sheep, rabbits, mice, rats, or *Chondrichthyes* (e.g., shark).

The term "humanized antibody" means an antibody obtained by grafting CDR sequences derived from another mammalian species, such as a mouse species, onto human framework sequences. To preserve the binding affinity, some residues in the backbone (referred to as FR) segments may be modified. The humanized antibodies or fragments thereof according to the present application can be prepared by techniques known to those skilled in the art.

The term "variable region" herein refers to a region of a heavy or light chain of an antibody involved in the binding of the antibody to an antigen. "Heavy chain variable region" is used interchangeably with "VH" and "HCVR", and "light chain variable region" is used interchangeably with "VL" and "LCVR". Heavy and light chain variable domains (VH and VL, respectively) of natural antibodies generally have similar structures, each of which contains four conservative framework regions (FRs) and three hypervariable regions (HVRs). See, e.g., Kindt et al., Kuby Immunology, 6th ed., W. H. Freeman and Co., p.91 (2007). A single VH or VL domain may be sufficient to provide antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region because it is precisely complementary to an epitope in a spatial structure, wherein the heavy chain variable region CDR may be abbreviated as HCDR and the light chain variable region CDR may be abbreviated as LCDR. The terms "framework region" or "FR" are used interchangeably and refer to those amino acid residues of an antibody heavy chain variable region or light chain variable region, other than CDRs. Generally, a typical antibody variable region consists of 4 FRs and 3 CDRs in the following order: FR1-CDR1-FR2-CDR2-FR3-CDR3-FR4.

For further description of the CDRs, see Kabat et al., J. Biol. Chem., 252: 6609-6616 (1977); Kabat et al., United States Department of Health and Human Services, Sequences of proteins of immunological interest (1991); Chothia et al., J. Mol. Biol. 196: 901-917 (1987); Al-Lazikani B. et al., J. Mol. Biol., 273: 927-948 (1997); MacCallum et al., J. Mol. Biol. 262: 732-745 (1996); Abhinandan and Martin, Mol. Immunol., 45: 3832-3839 (2008); Lefranc M.P. et al., Dev. Comp. Immunol., 27: 55-77 (2003); and Honegger and Plückthun, J. Mol. Biol., 309: 657-670 (2001). "CDR" herein may be labeled and defined in a manner well known in the art, including, but not limited to, Kabat numbering scheme; the tool sites used include, but are not limited to, abYsis site (www.abysis.org/abysis/sequence_input/key_annotation/key_annotation.cgi)).

The term "Kabat numbering scheme" herein generally refers to the immunoglobulin alignment and numbering scheme proposed by Elvin A. Kabat (see, e.g., Kabat et al., Sequences of Proteins of Immunological Interest, 5th Ed. Public Health Service, National Institutes of Health, Bethesda, Md., 1991).

As used herein, the terms "percent (%) sequence uniformity" and "percent (%) sequence identity" are used interchangeably, referring to the percentage of identity between amino acid (or nucleotide) residues of a candidate sequence and amino acid (or nucleotide) residues of a reference sequence after aligning the sequences and introducing gaps, if necessary, to achieve maximum percent sequence identity (e.g., gaps may be introduced in one or both of the candidate sequence and the reference sequence for optimal alignment, and non-homologous sequences may be omitted for the purpose of comparison). Alignment may be carried out in a variety of ways well known to those skilled in the art for the purpose of determining percent sequence identity, for example, using publicly available computer software such as BLAST, ALIGN, or Megalign (DNASTAIi) software. Those skilled in the art can determine appropriate parameters for measuring alignment, including any algorithms required to achieve maximum alignment of the full length of the aligned sequences. For example, a reference sequence aligned for comparison with a candidate sequence may show that the candidate sequence exhibits 50% to 100% sequence identity over the full length of the candidate sequence or a selected portion of contiguous amino acid (or nucleotide) residues of the candidate sequence. The length of the candidate sequence aligned for the purpose of comparison may be, e.g., at least 30% (e.g., 30%, 40%, 50%, 60%, 70%, 80%, 90%, or 100%) of the length of the reference sequence. When a position in the candidate sequence is occupied by the same amino acid (or nucleotide) residue at the corresponding position in the reference sequence, then the molecules are identical at that position.

As used herein, "mutation" includes insertion mutation, deletion mutation and substitution mutation, and in some embodiments, the substitution mutation is preferably a conservative amino acid substitution.

As used herein, "conservative amino acid" generally refers to amino acids that belong to the same class or have similar characteristics (e.g., charge, side chain size, hydrophobicity, hydrophilicity, backbone conformation, and rigidity). Illustratively, the amino acids in each of the following groups belong to conservative amino acid residues of each other, and substitutions of amino acid residues within the groups belong to conservative amino acid substitutions:
Illustratively, the following six groups are examples of amino acids that are considered to be conservative replacements of each other:
1) alanine (A), serine (S), and threonine (T);
2) aspartic acid (D) and glutamic acid (E);
3) asparagine (N) and glutamine (Q);
4) arginine (R), lysine (K), and histidine (H);
5) isoleucine (I), leucine (L), methionine (M), and valine (V); and
6) phenylalanine (F), tyrosine (Y), and tryptophan (W).

As used herein, "at most X mutations" means that the number of mutations may be selected from any natural number in the range of 0 to X.

As used herein, "at most 0-3 mutations" may be 3 mutations, 2 mutations, 1 mutation, or 0 mutations. As used herein, "respectively" of "the HCDR1-3 have at most 0-3 mutations, respectively", means that the mutation numbers per CDR in the HCDR1-3 are independent of each other and may each be independently selected from 0-3. Similarly, as used herein, "respectively" of "the LCDR1-3 have at most 0-3 mutations, respectively", means that the mutation numbers per CDR in the LCDR1-3 are independent of each other.

As used herein, "vector" is a composition that comprises an isolated nucleic acid and may be used for delivering the isolated nucleic acid to the interior of a cell. Many vectors are known in the art, including but not limited to, linear polynucleotides, polynucleotides associated with ionic or amphiphilic compounds, plasmids, and viruses. Thus, the term "vector" includes autonomously replicating plasmids or viruses. The term should also be construed as including non-plasmid and non-viral compounds that facilitate the transfer of nucleic acids into cells, such as polylysine compounds and liposomes. Examples of viral vectors include, but are not limited to, adenoviral vectors, adeno-associated viral vectors, retroviral vectors, etc.

As used herein, the terms "subject" and "patient" refer to an organism that receives treatment for a particular disease or disorder (e.g., a cancer or an infectious disease) as described herein. Examples of the subject and the patient include mammals, such as human, primate, pig, goat, rabbit, hamster, cat, dog, guinea pig, members of the bovine family (such as cattle, bison, buffalo, elk, yak, etc.), cattle, sheep, horse, bison, etc., that receive treatment for a disease or disorder (e.g., a cell proliferative disorder, such as a cancer or an infectious disease).

As used herein, the term "treatment" refers to surgical or therapeutic treatment for the purpose of preventing or slowing (reducing) the progression of an undesirable physiological or pathological change, such as a cell proliferative disorder (e.g., a cancer or an infectious disease), in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission (whether partial or total) of state of disease, whether detectable or undetectable. Subjects in need of treatment include those already with a disorder or disease, as well as those who are susceptible to a disorder or disease or those who intend to prevent a disorder or disease. When referring to terms such as slowing, alleviation, decrease, palliation, and remission, their meanings also include elimination, disappearance, nonoccurrence, etc.

As used herein, the term "effective amount" refers to an amount of a therapeutic agent that is effective in preventing or alleviating symptoms of a disease or the progression of the disease when administered to a cell, tissue or subject alone or in combination with another therapeutic agent. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers to the amount of the ingredient alone. When a combination is used, a therapeutically effective dose refers to the combined amounts of the active ingredients that produce the therapeutic effect, whether administered in combination, sequentially, or simultaneously.

The term "cancer" herein refers to or describes a physiological condition in mammals that is typically characterized by unregulated cell growth. Included in this definition are benign and malignant cancers. The term "tumor" or "neoplasm" herein refers to all neoplastic cell growth and proliferation, whether malignant or benign, and all pre-cancerous and cancerous cells and tissues. The terms "cancer" and "tumor" are not mutually exclusive when referred to herein.

The term "pharmaceutical composition" as used herein means a combination of at least one drug and optionally a pharmaceutically acceptable carrier or excipient combined together to achieve a certain objective. In certain embodiments, the pharmaceutical composition includes temporally and/or spatially separated combinations, so long as they can act together to achieve the objective of the present application. For example, the components contained in the pharmaceutical composition (e.g., the CAR-NK cell according to the present application) may be administered to an individual as a whole or separately. When administered to an individual separately, the components contained in the pharmaceutical composition may be administered to the individual simultaneously or sequentially. The pharmaceutical composition according to the present application may comprise conventional components of cell culture to maintain the activity of the CAR-NK cells. The pharmaceutically acceptable carrier may also include water, an aqueous buffer solution, an isotonic salt solution such as PBS (phosphate-buffered saline), glucose, mannitol, dextrose, lactose, starch, magnesium stearate, cellulose, magnesium carbonate, 0.3% glycerol, hyaluronic acid, ethanol, or a polyalkylene glycol such as polypropylene glycol, triglyceride, etc. The pharmaceutical composition or pharmaceutical formulation according to the present application may be administered by any suitable route, for example, intravenous administration, intradermal, subcutaneous, and intramuscular injection, etc. The composition according to the present application may comprise a wetting agent, an emulsifier or a buffer substance as an additive.

The inventors of the present application developed a novel chimeric antigen receptor targeting CD19 and use thereof through a great deal of research and tests, and provided a chimeric antigen receptor targeting CD19 and a corresponding nucleic acid molecule, a vector, an immune effector cell, a preparation method and a product thereof, a pharmaceutical composition, therapeutic use, pharmaceutical use, and a method for treating a tumor or cancer. Each invention of the present application realizes at least one of the following beneficial effects: (1) having a specific killing effect on CD19 positive tumor cells and/or having an inhibition effect on the growth of CD19-related tumors; (2) reducing the immunogenicity through humanizing the chimeric antigen receptor targeting CD19; (3) having application prospects for "shelf-type" therapeutic products in the case of an exemplary effector cell NK cell.

In a first aspect, the present application provides a nucleic acid molecule encoding a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 9-11, respectively, and the LCDR1-3 have the sequences set forth in SEQ ID NOs: 12-14, respectively; or
(2) the HCDR1-3 have the sequences set forth in SEQ ID NOs: 15-17, respectively, and the LCDR1-3 have the sequences set forth in SEQ ID NOs: 18-20, respectively; or
(3) the HCDR1-3 have the sequences set forth in SEQ ID NOs: 21-23, respectively, and the LCDR1-3 have the sequences set forth in SEQ ID NOs: 24-26, respectively; or
(4) the HCDR1-3 have the sequences set forth in SEQ ID NOs: 27-29, respectively, and the LCDR1-3 have the sequences set forth in SEQ ID NOs: 30-32, respectively; or
(5) the HCDR1-3 have at most 0-3 mutations (e.g., 0, 1, 2, and 3 mutations) compared with each corresponding CDR in any one of groups (1) - (4), respectively, and the LCDR1-3 have at most 0-3 mutations (e.g., 0, 1, 2, and 3 mutations) compared with each corresponding CDR in any one of groups (1) - (4), respectively.

In some embodiments, (1) the VH has the sequence set forth in SEQ ID NO: 1, and the VL has the sequence set forth in SEQ ID NO: 2; or (2) the VH has the sequence set forth in SEQ ID NO: 40, and the VL has the sequence set forth in SEQ ID NO: 39; or (3) the VH has the sequence set forth in SEQ ID NO: 41, and the VL has the sequence set forth in SEQ ID NO: 38; or (4) the VH has the sequence set forth in SEQ ID NO: 3, and the VL has the sequence set forth in SEQ ID NO: 4; (5) the VH has the sequence set forth in SEQ ID NO: 5, and the VL has the sequence set forth in SEQ ID NO: 6; (6) the VH has the sequence set forth in SEQ ID NO: 7 or 43, and the VL has the sequence set forth in SEQ ID NO: 8 or 42; or (7) the VH has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 25 mutations (e.g., at most 20 mutations, at most 15 mutations, at most 10 mutations, at most 5 mutations, at most 4 mutations, at most 3 mutations, at most 2 mutations, or 1 mutation) compared with the VH corresponding to any one of groups (1) - (6), and the VL has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 20 mutations (e.g., at most 15 mutations, at most 10 mutations, at most 5 mutations, at most 4 mutations, at most 3 mutations, at most 2 mutations, or 1 mutation) compared with the VL corresponding to any one of groups (1) - (6).

In some embodiments, the antigen-binding region is in the form of an scFv.

In some embodiments, the antigen-binding region in the form of an scFv has:
(1) the amino acid sequence set forth in SEQ ID NO: 51, 55, or 56; or
(2) the amino acid sequence set forth in SEQ ID NO: 52; or
(3) the amino acid sequence set forth in SEQ ID NO: 53; or
(4) the amino acid sequence set forth in SEQ ID NO: 54 or 57; or
(5) an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 50 mutations (e.g., at most 45 mutations, at most 40 mutations, at most 35 mutations, at most 30 mutations, at most 25 mutations, at most 20 mutations, at most 15 mutations, at most 10 mutations, at most 5 mutations, at most 4 mutations, at most 3 mutations, at most 2 mutations, or 1 mutation) compared with the amino acid sequence set forth in any one of SEQ ID NOs: 51-57.

EIVLTQSPTTMAASPGEKITITCSASSSISSNYLHWYQQKPGFSPKFLIYRTSNLASGVPARFS
GSGSGTSYSLTIGTMEAEDVATYYCQQASSIPRMFTFGSGTKLEIKGSTSGSGKPGSGEGST
KGQVQLQQPGAELVVPGTSVKLSCKASGYTFTNYWMHWVKQRPGQGLEWIGEIDPSDNY
ANYNQEFQGKATLTVDKSSSTAYMQLSSLTSDDSAVYYCARHDGYFGALDYWGQGTSVT
VSS (SEQ ID NO: 51, included in Mab01-CAR)

DVVLTQTPLSLPVSLGDQASISCRSSQSLENSNGNSYLNWYLQKPGQSPQLLIYRVSNRFSG
VLDRFSGSGSGTDFTLKISRVEAEDLGVYFCLQITHVPWTFGGGTKLEIKGSTSGSGKPGSG
EGSTKGEVQLQQSGPELVKPGASVKMSCKASGYTFTDYVMHWVRQTPGQGLEWIGYFNP

YNDGTNYNEKFKVKATLTSDKSSTTAYMELSSLTSEDSAVYYCARGVYYYGRDFDYWGQ
GTTLTVSS (SEQ ID NO: 52, included in Mab02-CAR)

DVVMTQTPLTLSVTIGQSASISCKSSQSLLESDGKTYLNWLLQRPGQSPKRLIYLVSKLDSG
VPDRFTGSASGTDFTLKISRVEAEDLGVYYCWQGTHFPWTFGGGTKLEIKGSTSGSGKPGS
GEGSTKGQVQLQQSGAELVRPGASVTLSCKASGYTFPDYEIHWVKQTPVHGLEWIGAIDPE
TGGIGYNQKFTGKAMLTADKSSSTAYMELRSLTSEDSAVYFCTRNYGSRWGQGSTLTVSS
(SEQ ID NO: 53, included in Mab03-CAR)

DIQMTQTTSSLSASLGDRVTISCRASQDISKYLNWYQQKPDGTVKLLIYHTSRLHSGVPSRF
SGSGSGTDYSLTISNLEQEDIATYFCQQGNTLPYTFGGGTKLEITGSTSGSGKPGSGEGSTKG
EVKLQESGPGLVAPSQSLSVTCTVSGVSLPDYGVSWIRQPPRKGLEWLGVIWGSETTYYNS
ALKSRLTIIKDNSKSQVFLKMNSLQTDDTAIYYCAKHYYYGGSYAMDYWGQGTSVTVSS
(SEQ ID NO: 54, included in FMC63-CAR)

EIVLTQSPATLSLSPGERATLSCSASSSISSNYLHWYQQKPGQAPRFLIYRTSNLASGIPARFS
GSGSGTDFTLTISSLEPEDFAVYYCQQASSIPRMFTFGQGTKLEIKGSTSGSGKPGSGEGSTK
GEVQLQQSGAEVKKPGASVKVSCKASGYTFTNYWMHWVRQRPGQGLEWMGEIDPSDNY
ANYNQEFQGRVTITVDKSASTAYMELSSLRSEDTAVYYCARHDGYFGALDYWGQGTTVT
VSS (SEQ ID NO: 55, included in Hab01-4-CAR)

EIVLTQSPDFQSVTPKEKVTITCSASSSISSNYLHWYQQKPDQSPKFLIYRTSNLASGVPSRFS
GSGSGTDFTLTINSLEAEDAATYYCQQASSIPRMFTFGQGTKLEIKGSTSGSGKPGSGEGST
KGEVQLVQSGAEVKKPGASVKVSCKASGYTFTNYWMHWVRQRPGQGLEWMGEIDPSDN
YANYNQEFQGRVTITVDKSASTAYMELSSLRSEDTAVYYCARHDGYFGALDYWGQGTTV
TVSS(SEQ ID NO:56, Hab01-11-CAR)

DIQMTQSPSSLSASVGDRVTITCRASQDISKYLNWYQQKPGGAVKLLIYHTSRLHSGVPSRF
SGSGSGTDYTLTISSLQPEDFATYYCQQGNTLPYTFGGGTKVEIKGSTSGSGKPGSGEGSTK
GEVTLKESGPVLVKPTETLTLTCTVSGFSLSDYGVSWIRQPPGKALEWLAVIWGSETTYYN
SALKSRLTISKDTSKSQVVLTMTNMDPVDTATYYCAKHYYYGGSYAMDYWGQGTTVTV
SS (SEQ ID NO: 57, included in hFMC63-CAR)

In some embodiments, the chimeric antigen receptor comprises a CD8α signal peptide, an antigen-binding region that specifically binds to CD19, a CD8 hinge region, a CD8 transmembrane region, a CD28 costimulatory domain, and CD3ζ.

In some embodiments, the CD8α signal peptide has the amino acid sequence set forth in SEQ ID NO: 33 or has an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 10 mutations (e.g., at most 5 mutations, at most 4 mutations, at most 3 mutations, at most 2 mutations, or 1 mutation) compared with the amino acid sequence set forth in SEQ ID NO: 33.
MALPVTALLLPLALLLHAARP (SEQ ID NO:33)

In some embodiments, the CD8α hinge region has the amino acid sequence set forth in SEQ ID NO: 47 or has an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 10 mutations (e.g., at most 5 mutations, at most 4 mutations, at most 3 mutations, at most 2 mutations, or 1 mutation) compared with the amino acid sequence set forth in SEQ ID NO: 47.
FVPVFLPAKRTTTPAPRPPTPAPTIASQPLSLRPEACRPAAGGAVHTRGLDFACD (SEQ ID NO:47)

In some embodiments, the CD8 transmembrane region has the amino acid sequence set forth in SEQ ID NO: 48 or has an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 10 mutations (e.g., at most 5 mutations, at most 4 mutations, at most 3 mutations, at most 2 mutations, or 1 mutation) compared with the amino acid sequence set forth in SEQ ID NO: 48.
IYIWAPLAGTCGVLLLSLVITLYC (SEQ ID NO:48)

In some embodiments, the CD3ζ has the amino acid sequence set forth in SEQ ID NO: 49 or has an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 10 mutations (e.g., at most 5 mutations, at most 4 mutations, at most 3 mutations, at most 2 mutations, or 1 mutation) compared with the amino acid sequence set forth in SEQ ID NO: 49.

RVKFSRSADAPAYQQGQNQLYNELNLGRREEYDVLDKRRGRDPEMGGKPRRKNPQEGLY
NELQKDKMAEAYSEIGMKGERRRGKGHDGLYQGLSTATKDTYDALHMQALPPR (SEQ ID
NO:49)

In some embodiments, the CD28 costimulatory domain has the amino acid sequence set forth in SEQ ID NO: 50 or has an amino acid sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 10 mutations (e.g., at most 5 mutations, at most 4 mutations, at most 3 mutations, at most 2 mutations, or 1 mutation) compared with the amino acid sequence set forth in SEQ ID NO: 50.
RSKRSRLLHSDYMNMTPRRPGPTRKHYQPYAPPRDFAAYRS (SEQ ID NO:50)

In some embodiments, the chimeric antigen receptor further comprises a cytokine linked by a self-cleaving peptide. In some embodiments, the cytokine is IL15. In some embodiments, the self-cleaving peptide is selected from F2A, T2A, P2A, and E2A.

In some embodiments, the chimeric antigen receptor has the sequence set forth in any one of SEQ ID NOs: 34-37 or 44-46, or has a sequence having at least 80% identity (e.g., at least 85%, at least 90%, at least 91%, at least 92%, at least 93%, at least 94%, at least 95%, at least 96%, at least 97%, at least 98%, at least 99%, or 100%) or at most 120 amino acid mutations (e.g., at most 110 mutations, at most 100 mutations, at most 90 mutations, at most 80 mutations, at most 70 mutations, at most 60 mutations, at most 50 mutations, at most 45 mutations, at most 40 mutations, at most 35 mutations, at most 30 mutations, at most 25 mutations, at most 20 mutations, at most 15 mutations, at most 10 mutations, at most 5 mutations, at most 4 mutations, at most 3 mutations, at most 2 mutations, or 1 mutation) compared with any one of SEQ ID NOs: 34-37 or 44-46.

In some embodiments, the nucleic acid molecule is DNA or RNA.

In some embodiments, the RNA is mRNA.

In some embodiments, the nucleic acid molecule is an isolated nucleic acid molecule. In some embodiments, the nucleic acid molecule is operably linked to a regulatory sequence that can be recognized by a host cell transformed with a vector.

In a second aspect, the present application provides a chimeric antigen receptor targeting CD19 encoded by the nucleic acid molecule according to the first aspect.

In a third aspect, the present application provides a vector (e.g., an expression vector) comprising the nucleic acid molecule according to the first aspect.

In a fourth aspect, the present application provides an immune effector cell, wherein the immune effector cell is introduced with the nucleic acid molecule according to the first aspect or is introduced with the vector according to the third aspect or expresses the chimeric antigen receptor according to the second aspect.

In some embodiments, the immune effector cell is an NK cell, a T cell, or an NKT cell. In some embodiments, the immune effector cell is an NK cell. In some embodiments, the NK cell is differentiated from an iPSC, or derived from peripheral blood or umbilical cord blood.

There are various techniques for culturing, expanding and obtaining NK cells *in vitro,* and the general principles and methodologies thereof are known to those skilled in the art.

In some embodiments, the NK cell is obtained from the *in vitro* culture and expansion of peripheral blood mononuclear cell (PBMC)-derived NK cells. PBMC is one of the main sources of NK cells, and has the advantages of relatively easy collection, easy *in vitro* expansion, no toxic and side effects, and the like. However, the proportion of NK cells in PBMCs is typically 10% to 15%, and methods for expanding PBMC-derived NK cells include the use of a combination of cytokines, feeder cells or membrane particles to stimulate the *in vitro* expansion of NK cells.

In some embodiments, the NK cell is obtained from the *in vitro* culture and expansion of umbilical cord blood-derived NK cells. There are generally two different methods for obtaining large quantities of NK cells from umbilical cord blood. One method is to expand NK cells in umbilical cord blood, and the other method is to induce differentiation of umbilical cord blood CD34+ hematopoietic stem/progenitor cells into NK cells, followed by expansion.

In some embodiments, the NK cell is obtained from the *in vitro* culture and expansion of an NK cell line. By way of example, NK-92 is the first NK cell-based immunotherapy to obtain FDA approval for clinical trials, and is a homogenous immortalized NK lymphoma cell.

In some embodiments, the NK cell is obtained from the *in vitro* induction, culture, and expansion of induced pluripotent stem cells (iPSCs) or mesenchymal stem cells (ESCs).

In a fifth aspect, the present application provides a method for preparing an immune effector cell, wherein the method comprises the steps of: providing an immune effector cell, and introducing the nucleic acid molecule according to the first aspect or the vector according to the third aspect into the immune effector cell. In some embodiments, the method further comprises culturing the immune effector cell after introducing the nucleic acid molecule or the vector.

In a sixth aspect, the present application provides a product prepared according to the method according to the fifth aspect.

In a seventh aspect, the present application provides a pharmaceutical composition comprising the nucleic acid molecule according to the first aspect or the vector according to the third aspect or the immune effector cell according to the fourth aspect or the product according to the sixth aspect, and a pharmaceutically acceptable carrier.

In some embodiments, the pharmaceutical composition is used for treating a tumor or cancer. In some embodiments, the tumor or cancer is a CD19-related tumor or cancer. In some embodiments, the tumor or cancer is a B cell malignancy. In some embodiments, the tumor or cancer is acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

In some embodiments, the CD19-related tumor or cancer means that a tumor cell or cancer cell surface expresses CD19. In some embodiments, the tumor is a tumor highly expressing CD19 (CD19+). In some embodiments, the tumor that highly expressing CD19 (CD19+) refers to a tumor cell population in which at least 60% of the tumor cells express CD19. In some embodiments, the tumor that highly expressing CD19 (CD19+) refers to a tumor cell population in which at least 70% of the tumor cells express CD19. In some embodiments, the tumor that highly expressing CD19 (CD19+) refers to a tumor cell population in which at least 80% of the tumor cells express CD19. In some embodiments, the tumor that highly expressing CD19 (CD19+) refers to a tumor cell population in which at least 90% of the tumor cells express CD19. In some embodiments, the tumor that highly expressing CD19 (CD19+) refers to a tumor cell population in which at least 95% of the tumor cells express CD19. In some embodiments, the tumor that highly expressing CD19 (CD19+) refers to a tumor cell population in which at least 98% of the tumor cells express CD19. In some embodiments, the tumor that highly expressing CD19 (CD19+) refers to a tumor cell population in which at least 99% of the tumor cells express CD19.

In an eighth aspect, the present application provides use of the nucleic acid molecule according to the first aspect or the vector according to the third aspect or the immune effector cell according to the fourth aspect or the product according to the sixth aspect or the pharmaceutical composition according to the seventh aspect in the manufacture of a medicament for treating a tumor or cancer. In some embodiments, the tumor or cancer is a CD19-related tumor or cancer. In some embodiments, the tumor or cancer is a B cell malignancy. In some embodiments, the tumor or cancer is acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

In a ninth aspect, the present application provides a method for treating cancer or a tumor, comprising administering to a subject in need an effective amount of the nucleic acid molecule according to the first aspect or the vector according to the third aspect or the immune effector cell according to the fourth aspect or the product according to the sixth aspect or the pharmaceutical composition according to the seventh aspect. In some embodiments, the tumor or cancer is a CD19-related tumor or cancer. In some embodiments, the tumor or cancer is a B cell malignancy. In some embodiments, the tumor or cancer is acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

It should be understood that the above detailed description is provided solely to enable those skilled in the art to understand the contents of the present application more clearly, and is not intended to be limiting in any way. Those skilled in the art are able to make various modifications and changes to the described embodiments.

### Example

The present application will be further described with reference to specific examples, and the advantages and features of the present application will become more apparent with the description. Experimental procedures without specified conditions in the examples are conducted according to conventional conditions or conditions recommended by the manufacturers. Reagents or instruments without specified manufacturers used herein are conventional products that are commercially available.

The examples of the present application are exemplary only, and do not limit the scope of the present application in any way. It will be understood by those skilled in the art that various modifications or substitutions may be made to the technical solutions of the present application in form and details without departing from the spirit and scope of the present application, and that these modifications and substitutions shall fall within the protection scope of the present application.

### Materials and methods

### SPR detection method

The anti-human CD19 antibody was captured using a Protein A chip (GE Healthcare; 29-127-558). The sample and running buffer was HBS-EP+ (10 mM HEPES, 150 mM NaCl, 3 mM EDTA, 0.05% surfactant P20) (GE Healthcare; BR-1006-69). The flow cell was set at 25 °C. The sample block was set at 16 °C. Both were pretreated with the running buffer. In each cycle, first, the antibody to be tested was captured using the Protein A chip, and then a single concentration of CD19 antigen protein was injected. The association and dissociation processes of the antibody with the antigen protein were recorded, and finally, the chip was regenerated using Glycine pH 1.5 (GE Healthcare; BR-1003-54). The association was determined by injecting different concentrations of recombinant human CD19-His in the solution and maintaining for 240 s, wherein the flow rate was 30 µL/min, and the protein was diluted in a 1:1 dilution ratio from 200 nM (see detailed results for actual concentrations tested) to obtain 5 concentrations in total. The dissociation phase was monitored for up to 600 s and triggered by switching from the sample solution to the running buffer. The surface was regenerated by washing with 10 mM glycine solution (pH 1.5) at a flow rate of 30 µL/min for 30 s. The difference in bulk refractive index was corrected by subtracting the responses obtained from the goat anti-human Fc surface. Blank injections were also subtracted (= double reference). The Langmuir 1:1 model was used to calculate the apparent KD and other kinetic parameters.

### Virus packaging

The day before virus packaging, 293T cells (purchased from ATCC) were digested with trypsin and inoculated onto 10 cm culture dishes at a density of 1E7 cells per culture dish. When the cells were transfected, the packaging plasmid and the target plasmid were mixed and then added into an α-MEM culture medium, and FuGENE^{®} HD transfection reagent (Promega, E2311) was added into another centrifuge tube containing the α-MEM culture medium. The diluted transfection reagent was dropwise added over the diluted plasmid, and the mixture was mixed well and left to stand at room temperature for 15 min. Finally, the mixture formed by the plasmid and the transfection reagent was added onto a 10 cm culture dish, shaken gently for 10 times, mixed well, and placed in an incubator. 3 days after cell transfection, the virus was harvested. 10 mL of the virus-containing culture supernatant was transferred to a 50 mL centrifuge tube and centrifuged at 4 °C at 1250 rpm for 5 min to remove dead 293T cells. The virus-containing supernatant was filtered, concentrated using Retro-X Concentrator concentration reagent (Clontech, 631455), aliquoted and stored at -80 °C for later use.

### NK cell purification and activation

NK cells were separated from monocytes. The purification was divided into two steps, namely removing CD3⁺ cells in the first step, and enriching CD56⁺ cells in the second step. Cell washing, CD3 magnetic bead incubation and resuspension were performed using Sepax, followed by removal of the CD3⁺ cells with CliniMACS. The CD3⁻ cell product after removal of CD3⁺ cells was subjected to washing, CD56 magnetic bead incubation and resuspension using Sepax again, and CD56⁺ cells were enriched with CliniMACS. The enriched CD56⁺ cells were washed with Sepax and exchanged into a complete medium. K562 feeder cells were added, and the mixture was transferred to a 37 °C incubator for 5-day activation.

### Virus transduction

On day 1, RetroNectin reagent was added to 24-well plates at 500 µL/well and coated at 4 °C overnight. On day 2, the upper-layer RetroNectin was discarded, and the plate was washed with PBS. Packaged retrovirus was added and centrifuged at 4-8 °C at 2000 g for 60 min. The upper-layer virus liquid was discarded. The NK cells activated for 5 days were added to 24-well plates at 3E5 cells/well, centrifuged at room temperature at 400 g for 5min, and cultured in a 37 °C incubator (37 °C, 5% CO₂). On day 3, the NK cells were transferred to 6-well plates without tissue culture treatment and cultured. On day 6, CAR expression was detected to ensure the CAR positive rate in the range of 60% to 80% for NK cells used for *in vitro* or *in vivo* killing.

### Determination of in vitro tumor cell killing rate

Unless otherwise specified, the target cells referred to in the following examples are stably transfected with a luciferase gene to express luciferase. The luciferase reporter gene assay reagent was used for determining the fluorescence intensity and reflecting the cell viability and the killing effect of NK cells. The experimental well killing rate calculation formula is as follows: killing rate = (target cell well reading - experimental well reading)/target cell well reading × 100%.

### Example 1. Preparation of CD19 Antibody

The mice were immunized with human CD19 protein (NCBI: NP_001761.3) as the immunogen. The mice in which the antibody titer in the serum was high and was reaching a plateau were selected. Spleen cells of the mice were fused with myeloma cells SP2/0 to give positive monoclonal hybridoma cells after culture and screening. The antibodies secreted by the positive monoclonal hybridoma cells were identified to give positive monoclonal hybridomas Mab01, Mab02 and Mab03 capable of secreting CD19 antibodies. The RNA of the positive monoclonal hybridoma cell was extracted, reversely transcribed into cDNA, and subjected to sequencing to give sequences of heavy chain and light chain variable regions of the hybridoma positive clonal antibodies. The information of the variable region sequences and their CDR (Kabat numbering scheme) sequences are shown in Tables 1 and 2.

The heavy chain variable region and the light chain variable region of Mab01, Mab02, Mab03, and positive control FMC63 were cloned into an expression vector comprising a signal peptide and a human IgG1 heavy chain constant region and an expression vector comprising a signal peptide and a human IgG1 light chain constant region. A human-murine chimeric antibody was expressed, and the binding ability of the chimeric antibody to human CD19 protein was detected by SPR (Biacore). The SPR detection results are shown in Table 3. Mab01-Mab03 all had good affinity for human CD19 protein.

**Table 1. Sequence information of antibodies**

| Sequence name | SEQ ID NO: | Sequence |
|---|---|---|
| Mab01-VH | 1 | QVQLQQPGAELVVPGTSVKLSCKASGYTFTNYWMHWVKQRPGQGLEWI GEIDPSDNYANYNQEFQGKATLTVDKSSSTAYMQLSSLTSDDSAVYYCA RHDGYFGALDYWGQGTSVTVSS |
| Mab01-VL | 2 | EIVLTQSPTTMAASPGEKITITCSASSSISSNYLHWYQQKPGFSPKFLIYRTS NLASGVPARFSGSGSGTSYSLTIGTMEAEDVATYYCQQASSIPRMFTFGSG TKLEIK |
| Mab02-VH | 3 | EVQLQQSGPELVKPGASVKMSCKASGYTFTDYVMHWVRQTPGQGLEWI GYFNPYNDGTNYNEKFKVKATLTSDKSSTTAYMELSSLTSEDSAVYYCA RGVYYYGRDFDYWGQGTTLTVSS |
| Mab02-VL | 4 | DVVLTQTPLSLPVSLGDQASISCRSSQSLENSNGNSYLNWYLQKPGQSPQ LLIYRVSNRFSGVLDRFSGSGSGTDFTLKISRVEAEDLGVYFCLQITHVPW TFGGGTKLEIK |
| Mab03-VH | 5 | QVQLQQSGAELVRPGASVTLSCKASGYTFPDYEIHWVKQTPVHGLEWIG AIDPETGGIGYNQKFTGKAMLTADKSSSTAYMELRSLTSEDSAVYFCTRN YGSRWGQGSTLTVSS |
| Mab03-VL | 6 | DVVMTQTPLTLSVTIGQSASISCKSSQSLLESDGKTYLNWLLQRPGQSPKR LIYLVSKLDSGVPDRFTGSASGTDFTLKISRVEAEDLGVYYCWQGTHFPW TFGGGTKLEIK |
| FMC63-VH | 7 | EVKLQESGPGLVAPSQSLSVTCTVSGVSLPDYGVSWIRQPPRKGLEWLGV IWGSETTYYNSALKSRLTIIKDNSKSQVFLKMNSLQTDDTAIYYCAKHYY YGGSYAMDYWGQGTSVTVSS |
| FMC63-VL | 8 | DIQMTQTTSSLSASLGDRVTISCRASQDISKYLNWYQQKPDGTVKLLIYHT SRLHSGVPSRFSGSGSGTDYSLTISNLEQEDIATYFCQQGNTLPYTFGGGT KLEIT |

**Table 2. CDRs corresponding to antibodies**

| Sequence name | CDR1 | CDR2 | CDR3 |
|---|---|---|---|
| Mab01-VH | NYWMH (SEQ ID NO:9) | EIDPSDNYANYNQEFQG (SEQ ID NO:10) | HDGYFGALDY (SEQ ID NO:11) |
| Mab01-VL | SASSSISSNYLH (SEQ ID NO:12) | RTSNLAS (SEQ ID NO:13) | QQASSIPRMFT (SEQ ID NO:14) |
| Mab02-VH | DYVMH (SEQ ID NO:15) | YFNPYNDGTNYNEKEKV (SEQ ID NO:16) | GVYYYGRDFDY (SEQ ID NO:17) |
| Mab02-VL | RSSQSLENSNGNSYLN (SEQ ID NO:18) | RVSNRFS (SEQ ID NO:19) | LQITHVPWT (SEQ ID NO:20) |
| Mab03-VH | DYEIH (SEQ ID NO:21) | AIDPETGGIGYNQKFTG (SEQ ID NO:22) | NYGSR (SEQ ID NO:23) |
| Mab03-VL | KSSQSLLESDGKTYLN (SEQ ID NO:24) | LVSKLDS (SEQ ID NO:25) | WQGTHFPWT (SEQ ID NO:26) |
| FMC63-VH | DYGVS (SEQ ID NO:27) | VIWGSETTYYNSALKS (SEQ ID NO:28) | HYYYGGSYAMDY (SEQ ID NO:29) |
| FMC63-VL | RASQDISKYLN (SEQ ID NO:30) | HTSRLHS (SEQ ID NO:31) | QQGNTLPYT (SEQ ID NO:32) |

**Table 3. Assay on affinity of chimeric antibodies for human CD19 by SPR (Biacore)**

| Antibody name | Ka (1/Ms) | Kd (1/s) | KD (M) |
|---|---|---|---|
| Mab01 | 3.27E+05 | 3.75E-04 | 1.15E-09 |
| Mab02 | 4.50E+05 | 1.11E-04 | 2.47E-10 |
| Mab03 | 5.39E+04 | 3.26E-04 | 6.05E-09 |
| FMC63 | 1.82E+05 | 3.33E-04 | 1.83E-09 |

### Example 2. Screening of Chimeric Antigen Receptor Targeting CD19

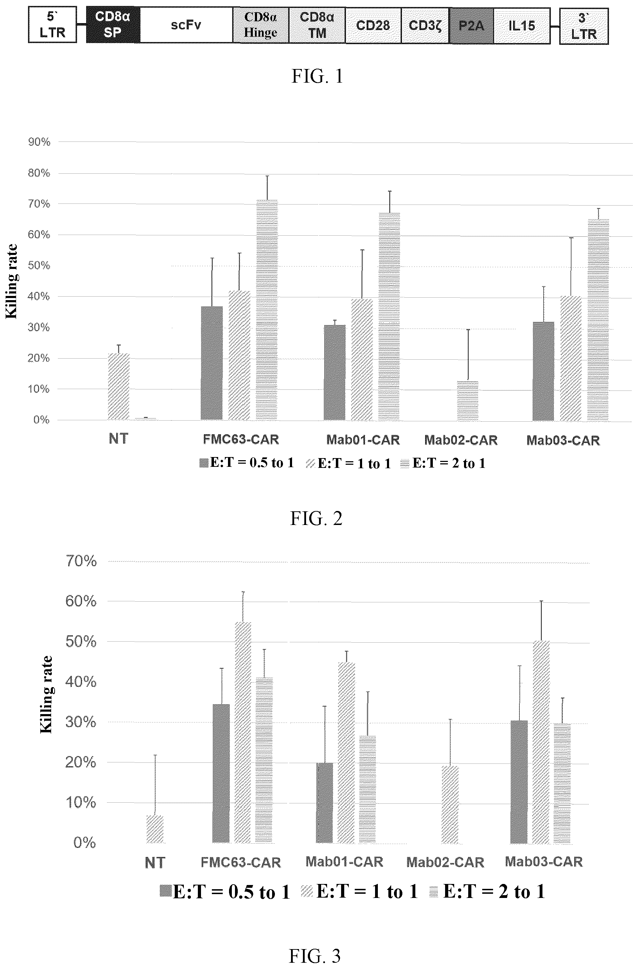

A chimeric antigen receptor targeting CD19 was constructed as shown in FIG. 1 using the anti-CD19 antibody screened in Example 1 and the positive control FMC63 antibody. Specifically, the chimeric antigen receptor targeting CD19 sequentially comprises a CD8α signal peptide (SP), an anti-CD19 scFv, a CD8 hinge region, a CD8 transmembrane region, a CD28 costimulatory domain, and CD3ζ, and IL15 linked by a self-cleaving peptide P2A. The specific sequences are detailed in Table 4. The nucleic acid molecule encoding the chimeric antigen receptor was cloned into a retroviral vector and, after packaging, used to transfect NK cells.

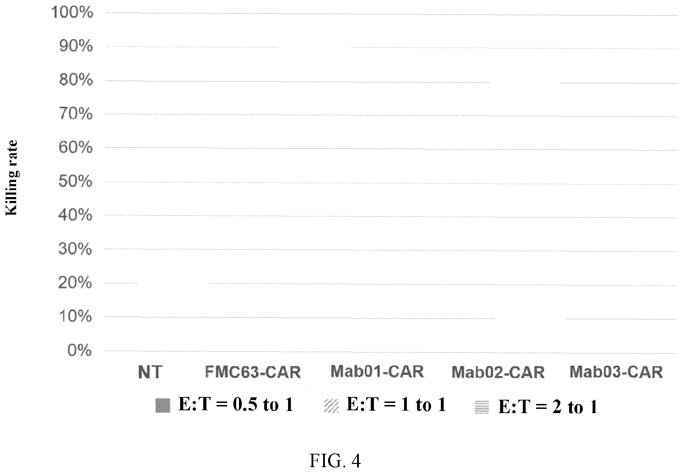

On day 6 after retroviral infection of NK cells, the specific killing of CD19-CAR NK cells on target cells Nalm6 and Raji that expressed CD19 was detected, and non-specific killing on target cells Molm13 that did not express CD19 was detected. 3 gradients were set for CAR NK-CD19 cells:target cells (effector-to-target ratio), which were 0.5:1, 1:1, and 2:1. The target cells were inoculated into a white opaque 96-well plate at 1E4 cells/well. NK cells were added according to the effector-to-target ratio, and the plate was cultured in an incubator at 37 °C with 5% CO₂ for 4 h. 30 µL of FIREFLYGLO luciferase reporter gene assay reagent (Meilunbio, MA0519-1) was added. The plate was incubated at room temperature for 10 min in the dark, and then measured with a microplate reader, and the killing rate was calculated. The detection results are shown in FIG. 2 to FIG. 4. The results show that FMC63-CAR, Mab01-CAR and Mab03-CAR had strong specific killing effect on CD19 positive cells (Nalm6 and Raji cells), and no significant difference existed in the killing effect, while FMC63-CAR, Mab01-CAR, Mab02-CAR and Mab03-CAR showed no non-specific killing against CD19 negative cells.

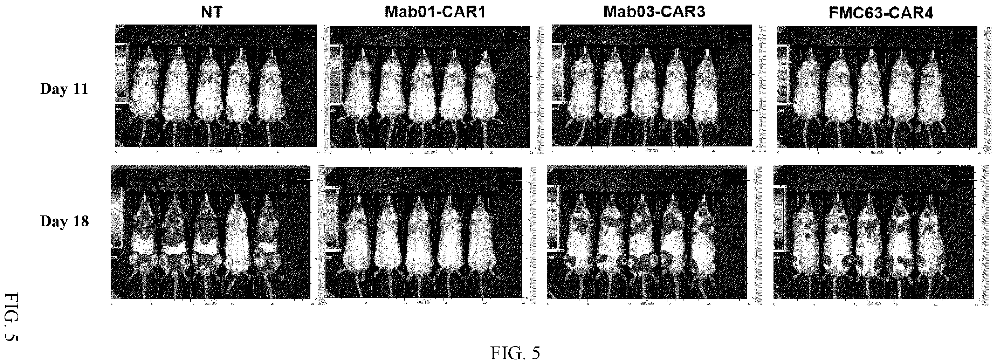

The tumor inhibition of Mab01-CAR and Mab03-CAR were further compared by a mouse tumor model. Specifically, NSG mice were weighed and randomly grouped according to Table 5, and human acute lymphocytic leukemia cells Nalm6 (carrying the luciferase gene) were inoculated at a cell amount of 0.25E6. A single dose of CD19 CAR NK cells (3E6 CAR-NK cells/mouse) was intravenously injected at a time and its inhibition effect on mouse tumor cell proliferation was observed. The results are shown in FIG. 5: Compared with NT (NK cells without transfected CD19-CAR), Mab01-CAR, Mab03-CAR and FMC63-CAR all showed tumor inhibition effect, and Mab01-CAR showed stronger tumor inhibition effect, which was significantly better than Mab03-CAR and FMC63-CAR. Mab01-CAR was selected for further development.

**Table 4. Sequence information of CD19 chimeric antigen receptors**

| Name | SEQ ID NO: | Sequence information |
|---|---|---|
| Mab01-CAR | 34 | MALPVTALLLPLALLLHAARPLEEIVLTQSPTTMAASPGEKITITCSASSSISSN YLHWYQQKPGFSPKFLIYRTSNLASGVPARFSGSGSGTSYSLTIGTMEAEDVA TYYCQQASSIPRMFTFGSGTKLEIKGSTSGSGKPGSGEGSTKGQVQLQQPGAE LVVPGTSVKLSCKASGYTFTNYWMHWVKQRPGQGLEWIGEIDPSDNYANYN QEFQGKATLTVDKSSSTAYMQLSSLTSDDSAVYYCARHDGYFGALDYWGQG TSVTVSSGSFVPVFLPAKRTTTPAPRPPTPAPTIASQPLSLRPEACRPAAGGAV HTRGLDFACDIYIWAPLAGTCGVLLLSLVITLYCRSKRSRLLHSDYMNMTPRR PGPTRKHYQPYAPPRDFAAYRSRVKFSRSADAPAYQQGQNQLYNELNLGRR EEYDVLDKRRGRDPEMGGKPRRKNPQEGLYNELQKDKMAEAYSEIGMKGE |
| | | RRRGKGHDGLYQGLSTATKDTYDALHMQALPPRGSGATNFSLLKQAGDVEE NPGPMRISKPHLRSISIQCYLCLLLNSHFLTEAGIHVFILGCFSAGLPKTEANWV NVISDLKKIEDLIQSMHIDATLYTESDVHPSCKVTAMKCFLLELQVISLESGDA SIHDTVENLIILANNSLSSNGNVTESGCKECEELEEKNIKEFLQSFVHIVQMFIN TS |
| Mab02-CAR | 35 | MALPVTALLLPLALLLHAARPLEDVVLTQTPLSLPVSLGDQASISCRSSQSLEN SNGNSYLNWYLQKPGQSPQLLIYRVSNRFSGVLDRFSGSGSGTDFTLKISRVE AEDLGVYFCLQITHVPWTFGGGTKLEIKGSTSGSGKPGSGEGSTKGEVQLQQS GPELVKPGASVKMSCKASGYTFTDYVMHWVRQTPGQGLEWIGYFNPYNDG TNYNEKFKVKATLTSDKSSTTAYMELSSLTSEDSAVYYCARGVYYYGRDFD YWGQGTTLTVSSGSFVPVFLPAKRTTTPAPRPPTPAPTIASQPLSLRPEACRPA AGGAVHTRGLDFACDIYIWAPLAGTCGVLLLSLVITLYCRSKRSRLLHSDYM NMTPRRPGPTRKHYQPYAPPRDFAAYRSRVKFSRSADAPAYQQGQNQLYNE LNLGRREEYDVLDKRRGRDPEMGGKPRRKNPQEGLYNELQKDKMAEAYSEI GMKGERRRGKGHDGLYQGLSTATKDTYDALHMQALPPRGSGATNFSLLKQ AGDVEENPGPMRISKPHLRSISIQCYLCLLLNSHFLTEAGIHVFILGCFSAGLPK TEANWVNVISDLKKIEDLIQSMHIDATLYTESDVHPSCKVTAMKCFLLELQVI SLESGDASIHDTVENLIILANNSLSSNGNVTESGCKECEELEEKNIKEFLQSFVH IVQMFINTS |
| Mab03-CAR | 36 | MALPVTALLLPLALLLHAARPLEDVVMTQTPLTLSVTIGQSASISCKSSQSLLE SDGKTYLNWLLQRPGQSPKRLIYLVSKLDSGVPDRFTGSASGTDFTLKISRVE AEDLGVYYCWQGTHFPWTFGGGTKLEIKGSTSGSGKPGSGEGSTKGQVQLQ QSGAELVRPGASVTLSCKASGYTFPDYEIHWVKQTPVHGLEWIGAIDPETGGI GYNQKFTGKAMLTADKSSSTAYMELRSLTSEDSAVYFCTRNYGSRWGQGST LTVSSGSFVPVFLPAKRTTTPAPRPPTPAPTIASQPLSLRPEACRPAAGGAVHT RGLDFACDIYIWAPLAGTCGVLLLSLVITLYCRSKRSRLLHSDYMNMTPRRPG PTRKHYQPYAPPRDFAAYRSRVKFSRSADAPAYQQGQNQLYNELNLGRREE YDVLDKRRGRDPEMGGKPRRKNPQEGLYNELQKDKMAEAYSEIGMKGERR RGKGHDGLYQGLSTATKDTYDALHMQALPPRGSGATNFSLLKQAGDVEENP GPMRISKPHLRSISIQCYLCLLLNSHFLTEAGIHVFILGCFSAGLPKTEANWVN VISDLKKIEDLIQSMHIDATLYTESDVHPSCKVTAMKCFLLELQVISLESGDASI HDTVENLIILANNSLSSNGNVTESGCKECEELEEKNIKEFLQSFVHIVQMFINTS |
| FMC63-CAR | 37 | MALPVTALLLPLALLLHAARPLEDIQMTQTTSSLSASLGDRVTISCRASQDISK YLNWYQQKPDGTVKLLIYHTSRLHSGVPSRFSGSGSGTDYSLTISNLEQEDIA TYFCQQGNTLPYTFGGGTKLEITGSTSGSGKPGSGEGSTKGEVKLQESGPGLV APSQSLSVTCTVSGVSLPDYGVSWIRQPPRKGLEWLGVIWGSETTYYNSALKS RLTIIKDNSKSQVFLKMNSLQTDDTAIYYCAKHYYYGGSYAMDYWGQGTSV TVSSGSFVPVFLPAKRTTTPAPRPPTPAPTIASQPLSLRPEACRPAAGGAVHTR GLDFACDIYIWAPLAGTCGVLLLSLVITLYCRSKRSRLLHSDYMNMTPRRPGP TRKHYQPYAPPRDFAAYRSRVKFSRSADAPAYQQGQNQLYNELNLGRREEY DVLDKRRGRDPEMGGKPRRKNPQEGLYNELQKDKMAEAYSEIGMKGERRR GKGHDGLYQGLSTATKDTYDALHMQALPPRGSGATNFSLLKQAGDVEENPG PMRISKPHLRSISIQCYLCLLLNSHFLTEAGIHVFILGCFSAGLPKTEANWVNVI SDLKKIEDLIQSMHIDATLYTESDVHPSCKVTAMKCFLLELQVISLESGDASIH DTVENLIILANNSLSSNGNVTESGCKECEELEEKNIKEFLQSFVHIVQMFINTS |

**Table 5. Grouping of NSG mice**

| Group | Number of mice | NK cell | Dose | Volume | Vehicle |
|---|---|---|---|---|---|
| 1 | 5 | Blank NK | 3E6 | 200µl | PBS |
| 2 | 5 | Mab01-CAR | 3E6 | 200µl | PBS |
| 3 | 5 | Mab03-CAR | 3E6 | 200µl | PBS |
| 4 | 5 | FMC63-CAR | 3E6 | 200µl | PBS |

### Example 3. Humanization of Murine Monoclonal Antibody Mab01

To reduce the immunogenicity of Mab01-CAR, Mab01 was humanized. Specifically, by alignment with the IMGT database (http://imgt.cines.fr) for germline genes from heavy and light chain variable regions of human antibodies, heavy chain variable region germline genes IGHV1-3*01 and IGHJ6*01 and light chain variable region germline genes IGKV6-21*01/IGKV3-11*01 and IGKJ2*01, with high homology to the murine antibody Mab01, were selected as templates, and CDRs of the murine antibodies were separately grafted into corresponding human templates to give variable region sequences in the order of FR1-CDR1-FR2-CDR2-FR3-CDR3-FR4. Key amino acids in a framework sequence were back-mutated to amino acids corresponding to the murine antibody as needed to ensure the original affinity (if there are sites in the antibody that are susceptible to chemical modification, mutations were performed at these sites to eliminate modification risks). The amino acid residues of the CDRs of the antibodies were identified by the Kabat numbering scheme. The sequence information of the humanized VH and VL is shown in Table 6, and the resulting humanized antibody variable region combinations are shown in Table 7.

The heavy chain variable region and the light chain variable region of Hab01-4 and Hab01-11 were cloned into an expression vector comprising a signal peptide and a murine IgG1 heavy chain constant region and an expression vector comprising a signal peptide and a murine IgG1 light chain constant region. An antibody in which the variable region was humanized was expressed, and the binding ability of the chimeric antibody to human CD19 protein was detected by SPR (Biacore), which is detailed in Table 8. Hab01-4 and Hab01-11 both had strong binding ability to the human CD19 protein.

FMC63 was subjected to humanization design in the same way. The humanized antibody hFMC63 was constructed and expressed, and the binding ability of the antibody to human CD19 protein was detected by SPR (Biacore). For details, see Table 6 and Table 9. hFMC63 had strong binding ability to human CD19.

**Table 6. Sequence information of humanized antibodies of Mab01 and FMC63**

| Sequence name | SEQ ID NO: | Sequence |
|---|---|---|
| Hab01-VL1 | 38 | EIVLTQSPATLSLSPGERATLSCSASSSISSNYLHWYQQKPGQAPRFLIYRT SNLASGIPARFSGSGSGTDFTLTISSLEPEDFAVYYCQQASSIPRMFTFGQG TKLEIK |
| Hab01-VL3 | 39 | EIVLTQSPDFQSVTPKEKVTITCSASSSISSNYLHWYQQKPDQSPKFLIYRT SNLASGVPSRFSGSGSGTDFTLTINSLEAEDAATYYCQQASSIPRMFTFGQ GTKLEIK |
| Hab01-VH3 | 40 | EVQLVQSGAEVKKPGASVKVSCKASGYTFTNYWMHWVRQRPGQGLEW MGEIDPSDNYANYNQEFQGRVTITVDKSASTAYMELSSLRSEDTAVYYC ARHDGYFGALDYWGQGTTVTVSS |
| Hab01-VH4 | 41 | EVQLQQSGAEVKKPGASVKVSCKASGYTFTNYWMHWVRQRPGQGLEW MGEIDPSDNYANYNQEFQGRVTITVDKSASTAYMELSSLRSEDTAVYYC ARHDGYFGALDYWGQGTTVTVSS |
| hFMC63-VL1 | 42 | DIQMTQSPSSLSASVGDRVTITCRASQDISKYLNWYQQKPGGAVKLLIYH TSRLHSGVPSRFSGSGSGTDYTLTISSLQPEDFATYYCQQGNTLPYTFGGG TKVEIK |
| hFMC63-VH1 | 43 | EVTLKESGPVLVKPTETLTLTCTVSGFSLSDYGVSWIRQPPGKALEWLAVI WGSETTYYNSALKSRLTISKDTSKSQVVLTMTNMDPVDTATYYCAKHYY YGGSYAMDYWGQGTTVTVSS |

**Table 7. Mab01 humanized antibody variable region combinations**

| Fv | Hab01-VH3 | Hab01-VH4 |
|---|---|---|
| Hab01-VL1 | / | Hab01-4 |
| Hab01-VL3 | Hab01-11 | / |

**Table 8. Assay on affinity of Mab01 humanized antibody for human CD19 by SPR (Biacore)**

| Antibody name | Ka (1/Ms) | Kd (1/s) | KD (M) |
|---|---|---|---|
| Hab01-4 | 9.11E+04 | 1.86E-08 | 2.05E-13 |
| Hab01-11 | 1.18E+05 | 1.04E-06 | 8.80E-12 |

**Table 9. Assay on affinity of FMC63 humanized antibody for human CD19 by SPR (Biacore)**

| Antibody name | Ka (1/Ms) | Kd (1/s) | KD (M) |
|---|---|---|---|
| hFMC63 | 9.24E+04 | 1.80E-06 | 1.95E-11 |
| FMC63 | 9.53E+04 | 1.17E-04 | 1.23E-09 |

### Example 4. Screening of Humanized Chimeric Antigen Receptor Targeting CD19

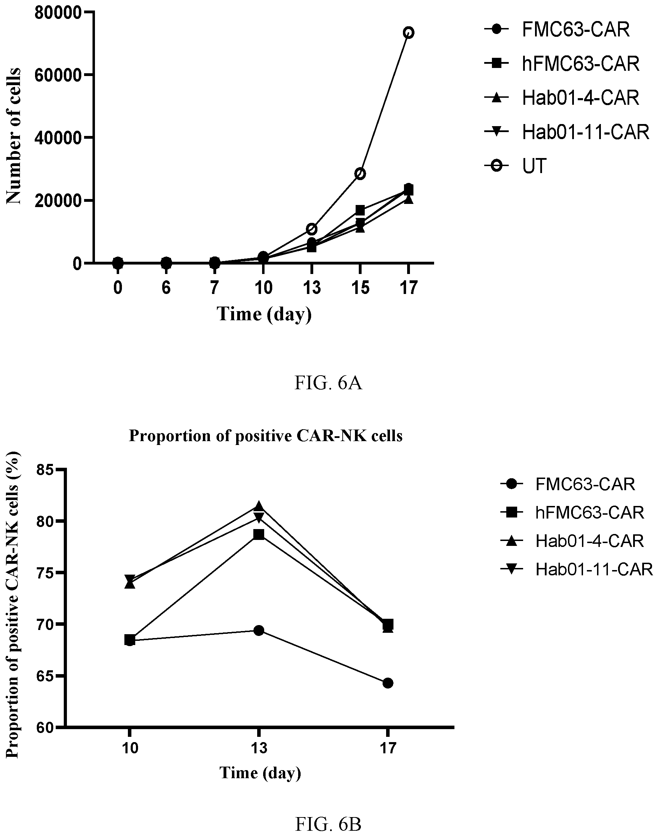

The humanized antibodies Hab01-4, Hab01-11 and hFMC 63 prepared in Example 3 were loaded into the chimeric antigen receptor shown in FIG. 1 to construct a humanized chimeric antigen receptor targeting CD19 (see Table 10 for specific sequence information), and the nucleic acid molecule encoding the chimeric antigen receptor was cloned into a retroviral vector and, after virus packaging, transfected into umbilical cord blood-derived NK cells. The NK cell expansion and CAR positive rate after transfection were determined, and the results are detailed in FIGs. 6A-6B. As shown in FIG. 6A, NK cells transfected with different CAR structures were substantially comparable in expansion ability; as shown in FIG. 6B, Hab01-4-CAR and Hab01-11-CAR had the highest CAR positive rate, hFMC63-CAR was slightly poor, and FMC63-CAR had the lowest positive rate, but the positive rates were about 60-80%, which met the requirements. On day 6 after retroviral infection of NK cells, sufficient cells were collected and cryopreserved.

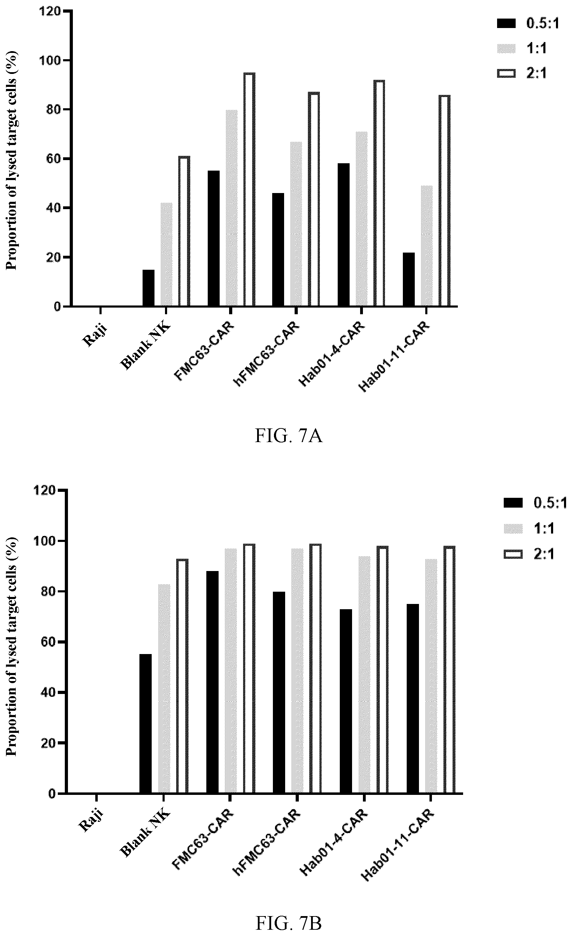

Before the experiment for detecting the killing activity on the target cells, the cryopreserved CAR-NK cells were thawed, and the CD19-CAR NK cells with the same number were respectively taken to be mixed with the target cells Raji at the beginning of thawing (0 h) and 24 h after thawing. The *in vitro* killing effect of the NK cells on the target cells under different effector-to-target ratios (Raji: 1E6 cells/mL) was detected, and the results are shown in FIGs. 7A-7B. FMC63-CAR, hFMC63-CAR, Hab01-4-CAR and Hab01-11-CAR which were just thawed (FIG. 7A) and 24 h after thawing (FIG. 7B) had strong killing effect on Raji cells; in contrast, after 24 h of thawing, the killing effect of CAR-NK cells was better.

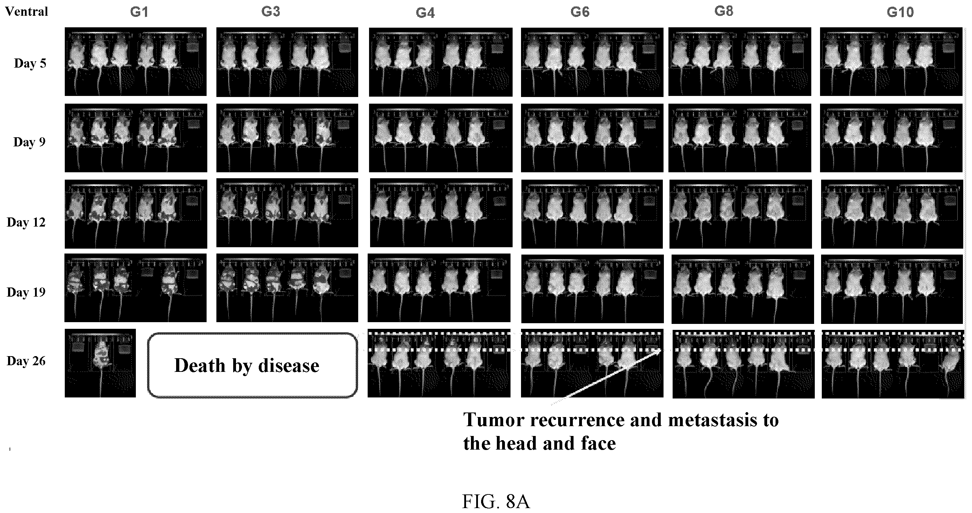

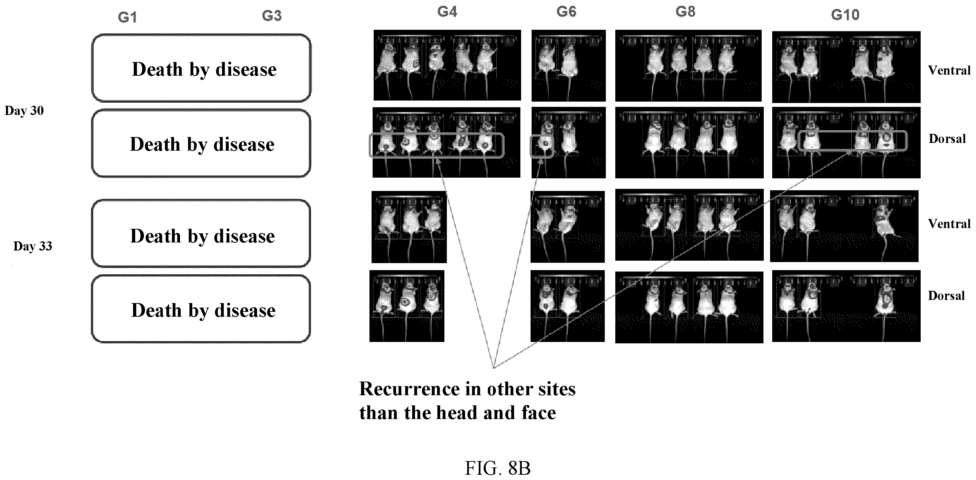

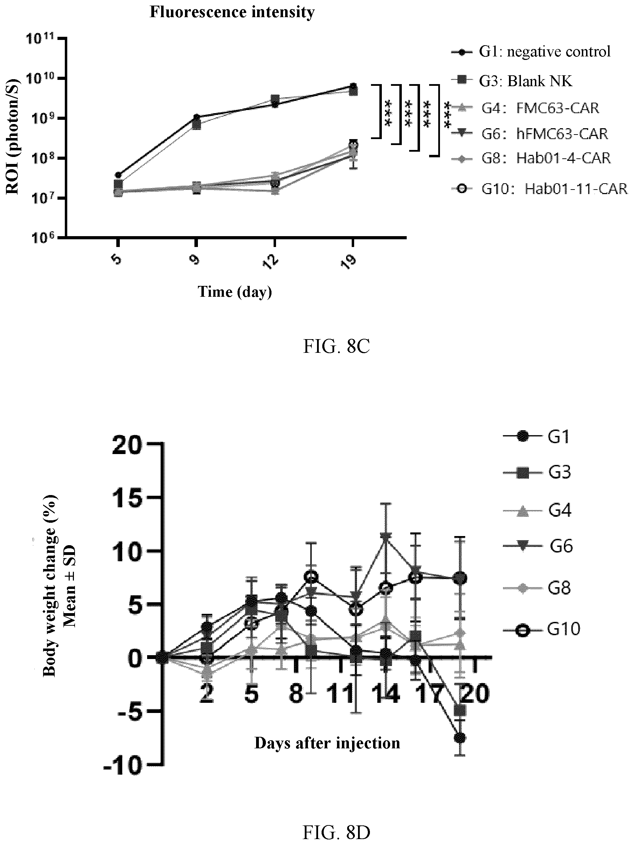

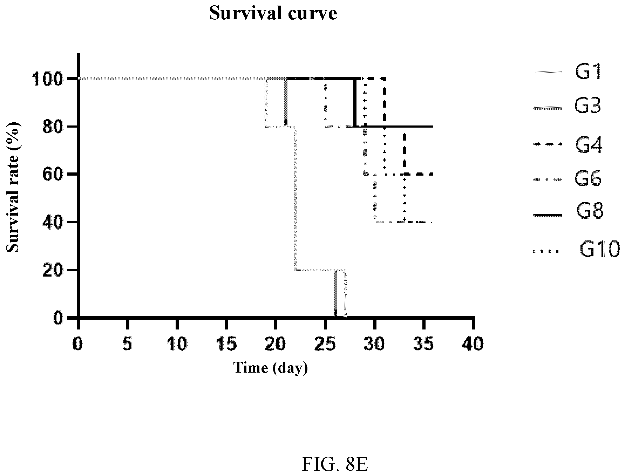

A Nalm6 xenograft mouse model was constructed to evaluate the tumor inhibition of FMC63-CAR, hFMC63-CAR, Hab01-4-CAR, and Hab01-11-CAR. Specifically, NOG mice were weighed, randomly grouped (see Table 11 for the grouping protocol), injected with human acute lymphocytic leukemia Nalm6 (carrying the luciferase gene) at a dose of 2.5E6 cells/mouse, and intravenously injected with a single dose of CD19 CAR NK cells (4E6 CAR-NK cells/mouse) 2 days later. The time point for injection of CD19 CAR NK cells was defined as day 0. The *in vivo* tumor development (fluorescence imaging), mouse body weight and survival status of the mice in groups G1, G3, G4, G6, G8, and G10 were continuously observed. The ratio of CAR19⁺CD56⁺ to CAR19⁻CD56⁺ was determined by taking peripheral blood from the mice in groups G2, G5, G7, G9 and G11 on days 0, 7, 16 and 21, and the blood routine examination and blood biochemistry indexes of the peripheral blood were determined on days 0, 7, 14 and 28. The results are shown in FIG. 8A to FIG. 8E, FIG. 9A to FIG. 9B, and FIG. 10.

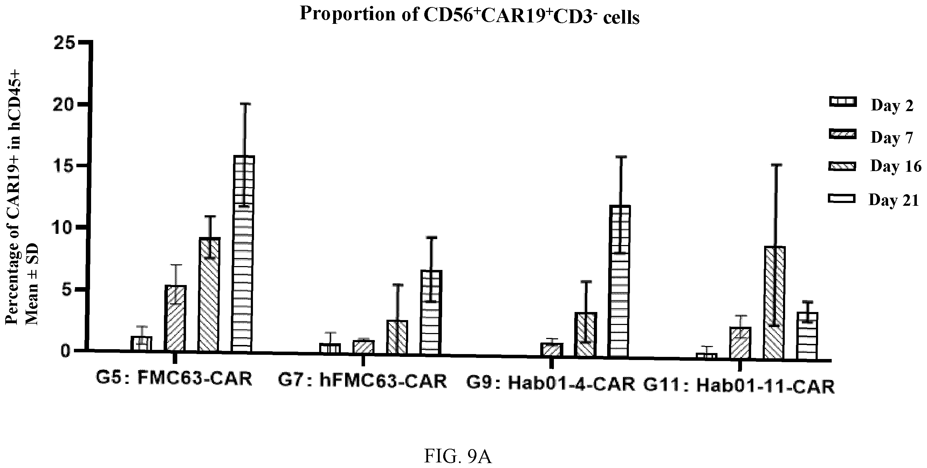

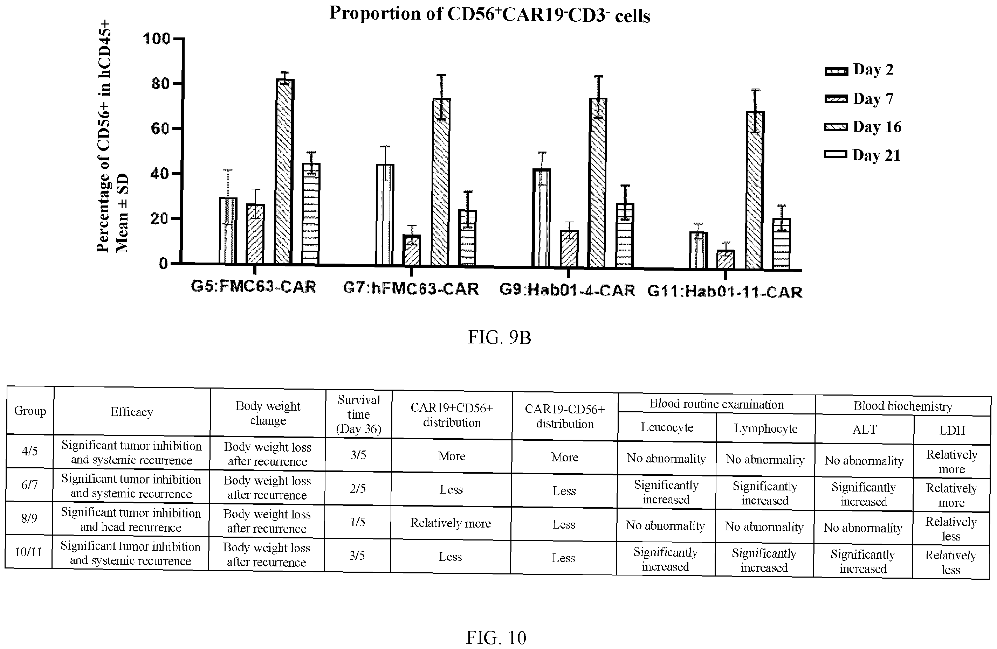

As shown in FIG. 8A to FIG. 8E, the tumor signals of the negative control groups (G1 and G3) continued to increase after tumor bearing, and the 4 CD19CAR NK treatment groups (G4, G6, G8 and G10) all showed significant antitumor efficacy, which significantly prolonged the survival time of the mice. In the 4 CD19 CAR NK treatment groups, the Hab01-4-CAR NK treatment group (group G8) had the best therapeutic effect. Although group G8 had recurrence of tumor metastasis to the head and face on day 26 as the other treatment groups, it was found that group G8 had no recurrence at other sites during days 30-33 by dorsal and ventral imaging observations, while the other treatment groups had recurrence at other sites than the head and face. Meanwhile, from the perspective of survival curve, the survival rate of the mice in group G8 was 80% (day 36), which was superior to that of the other treatment groups. In terms of body weight change, the 4 CD19 CAR-NK treatment group had well-conditioned mice without treatment-related toxicity such as body weight loss before day 19. Body weight loss began to appear on day 19, while fluorescence signals were detected at the mandible. The body weight loss of the mice was caused by tumor recurrence. As shown in FIG. 9A to FIG. 9B, in terms of the CD19 CAR⁺CD56⁺ proportion, group G5 > group G9 > group G11 > group G7; in terms of the CD19 CAR⁻CD56⁺ proportion, group G11 < group G9 = group G7 < group G5; in the G5, G7, G9, and G11 groups using humanized antibodies, group G9 had a higher relative number of the CAR⁺NK cells in the peripheral blood mononuclear cells. The blood routine examination and blood biochemistry indexes are detailed in FIG. 10. In group G9, the levels of alanine transaminase (ALT) and lactate dehydrogenase (LDH) were lower relative to the other CD19 CAR NK treatment groups, indicating a greater safety overall.

**Table 10. Sequence information of humanized chimeric antigen receptor targeting CD19**

| Name | SEQ ID NO: | Sequence information |
|---|---|---|
| Hab01-4-CAR | 44 | MALPVTALLLPLALLLHAARPLEEIVLTQSPATLSLSPGERATLSCSASSSISS NYLHWYQQKPGQAPRFLIYRTSNLASGIPARFSGSGSGTDFTLTISSLEPEDF AVYYCQQASSIPRMFTFGQGTKLEIKGSTSGSGKPGSGEGSTKGEVQLQQSG AEVKKPGASVKVSCKASGYTFTNYWMHWVRQRPGQGLEWMGEIDPSDNY ANYNQEFQGRVTITVDKSASTAYMELSSLRSEDTAVYYCARHDGYFGALD YWGQGTTVTVSSGSFVPVFLPAKRTTTPAPRPPTPAPTIASQPLSLRPEACRP AAGGAVHTRGLDFACDIYIWAPLAGTCGVLLLSLVITLYCRSKRSRLLHSDY MNMTPRRPGPTRKHYQPYAPPRDFAAYRSRVKFSRSADAPAYQQGQNQLY NELNLGRREEYDVLDKRRGRDPEMGGKPRRKNPQEGLYNELQKDKMAEA YSEIGMKGERRRGKGHDGLYQGLSTATKDTYDALHMQALPPRGSGATNFS LLKQAGDVEENPGPMRISKPHLRSISIQCYLCLLLNSHFLTEAGIHVFILGCFS AGLPKTEANWVNVISDLKKIEDLIQSMHIDATLYTESDVHPSCKVTAMKCF LLELQVISLESGDASIHDTVENLIILANNSLSSNGNVTESGCKECEELEEKNIK EFLQSFVHIVQMFINTS |
| Hab01-11-CAR | 45 | MALPVTALLLPLALLLHAARPLEEIVLTQSPDFQSVTPKEKVTITCSASSSISS NYLHWYQQKPDQSPKFLIYRTSNLASGVPSRFSGSGSGTDFTLTINSLEAED AATYYCQQASSIPRMFTFGQGTKLEIKGSTSGSGKPGSGEGSTKGEVQLVQS GAEVKKPGASVKVSCKASGYTFTNYWMHWVRQRPGQGLEWMGEIDPSDN YANYNQEFQGRVTITVDKSASTAYMELSSLRSEDTAVYYCARHDGYFGAL DYWGQGTTVTVSSGSFVPVFLPAKRTTTPAPRPPTPAPTIASQPLSLRPEACR PAAGGAVHTRGLDFACDIYIWAPLAGTCGVLLLSLVITLYCRSKRSRLLHSD YMNMTPRRPGPTRKHYQPYAPPRDFAAYRSRVKFSRSADAPAYQQGQNQL YNELNLGRREEYDVLDKRRGRDPEMGGKPRRKNPQEGLYNELQKDKMAE AYSEIGMKGERRRGKGHDGLYQGLSTATKDTYDALHMQALPPRGSGATNF SLLKQAGDVEENPGPMRISKPHLRSISIQCYLCLLLNSHFLTEAGIHVFILGCF SAGLPKTEANWVNVISDLKKIEDLIQSMHIDATLYTESDVHPSCKVTAMKCF LLELQVISLESGDASIHDTVENLIILANNSLSSNGNVTESGCKECEELEEKNIK EFLQSFVHIVQMFINTS |
| hFMC63-CAR | 46 | MALPVTALLLPLALLLHAARPLEDIQMTQSPSSLSASVGDRVTITCRASQDIS KYLNWYQQKPGGAVKLLIYHTSRLHSGVPSRFSGSGSGTDYTLTISSLQPED FATYYCQQGNTLPYTFGGGTKVEIKGSTSGSGKPGSGEGSTKGEVTLKESGP VLVKPTETLTLTCTVSGFSLSDYGVSWIRQPPGKALEWLAVIWGSETTYYNS ALKSRLTISKDTSKSQVVLTMTNMDPVDTATYYCAKHYYYGGSYAMDYW GQGTTVTVSSGSFVPVFLPAKRTTTPAPRPPTPAPTIASQPLSLRPEACRPAA GGAVHTRGLDFACDIYIWAPLAGTCGVLLLSLVITLYCRSKRSRLLHSDYM NMTPRRPGPTRKHYQPYAPPRDFAAYRSRVKFSRSADAPAYQQGQNQLYN ELNLGRREEYDVLDKRRGRDPEMGGKPRRKNPQEGLYNELQKDKMAEAY SEIGMKGERRRGKGHDGLYQGLSTATKDTYDALHMQALPPRGSGATNFSL LKQAGDVEENPGPMRISKPHLRSISIQCYLCLLLNSHFLTEAGIHVFILGCFSA GLPKTEANWVNVISDLKKIEDLIQSMHIDATLYTESDVHPSCKVTAMKCFLL ELQVISLESGDASIHDTVENLIILANNSLSSNGNVTESGCKECEELEEKNIKEF LQSFVHIVQMFINTS |

**Table 11. Grouping of NOG mice**

| Group | | Number of mice | NK cell | Dose | Volume | Vehicle |
|---|---|---|---|---|---|---|
| G1 | Efficacy | 5 | Negative control | / | 200µl | PBS |
| G2 | Metabolism/toxicology | 5 | Negative control | 4E6 | 200µl | PBS |
| G3 | Efficacy | 5 | Blank NK | 4E6 | 200µl | PBS |
| G4 | Efficacy | 5 | FMC63-CAR | 4E6 | 200µl | PBS |
| G5 | Metabolism/toxicology | 20 | FMC63-CAR | 4E6 | 200µl | PBS |
| G6 | Efficacy | 5 | hFMC63-CAR | 4E6 | 200µl | PBS |
| G7 | Metabolism/toxicology | 20 | hFMC63-CAR | 4E6 | 200µl | PBS |
| G8 | Efficacy | 5 | Hab01-4-CAR | 4E6 | 200µl | PBS |
| G9 | Metabolism/toxicology | 20 | Hab01-4-CAR | 4E6 | 200µl | PBS |
| G10 | Efficacy | 5 | Hab01-11-CAR | 4E6 | 200µl | PBS |
| G11 | Metabolism/toxicology | 20 | Hab01-11-CAR | 4E6 | 200µl | PBS |

The teachings of all patents, published applications and references cited herein are incorporated by reference in their entirety.

While exemplary examples have been specifically shown and described, it will be understood by those skilled in the art that various changes in forms and details may be made therein without departing from the scope of the embodiments encompassed by the appended claims.

## Claims

1. A nucleic acid molecule encoding a chimeric antigen receptor targeting CD19, wherein the chimeric antigen receptor comprises: an extracellular region comprising an antigen-binding region that specifically binds to CD19, a transmembrane region linked to the extracellular region, and an intracellular domain linked to the transmembrane region, and the antigen-binding region comprises a VH comprising HCDR1-3 and a VL comprising LCDR1-3, wherein:
(1) the HCDR1-3 have the sequences set forth in SEQ ID NOs: 9-11, respectively, and the LCDR1-3 have the sequences set forth in SEQ ID NOs: 12-14, respectively; or
(2) the HCDR1-3 have the sequences set forth in SEQ ID NOs: 15-17, respectively, and the LCDR1-3 have the sequences set forth in SEQ ID NOs: 18-20, respectively; or
(3) the HCDR1-3 have the sequences set forth in SEQ ID NOs: 21-23, respectively, and the LCDR1-3 have the sequences set forth in SEQ ID NOs: 24-26, respectively; or
(4) the HCDR1-3 have the sequences set forth in SEQ ID NOs: 27-29, respectively, and the LCDR1-3 have the sequences set forth in SEQ ID NOs: 30-32, respectively; or
(5) the HCDR1-3 have 0-3 mutations compared with each corresponding CDR in any one of groups (1) - (4), respectively, and the LCDR1-3 have 0-3 mutations compared with each corresponding CDR in any one of groups (1) - (4), respectively.

2. The nucleic acid molecule according to claim 1, wherein:
(1) the VH has the sequence set forth in SEQ ID NO: 1, and the VL has the sequence set forth in SEQ ID NO: 2; or
(2) the VH has the sequence set forth in SEQ ID NO: 40, and the VL has the sequence set forth in SEQ ID NO: 39; or
(3) the VH has the sequence set forth in SEQ ID NO: 41, and the VL has the sequence set forth in SEQ ID NO: 38; or
(4) the VH has the sequence set forth in SEQ ID NO: 3, and the VL has the sequence set forth in SEQ ID NO: 4;
(5) the VH has the sequence set forth in SEQ ID NO: 5, and the VL has the sequence set forth in SEQ ID NO: 6;
(6) the VH has the sequence set forth in SEQ ID NO: 7 or 43, and the VL has the sequence set forth in SEQ ID NO: 8 or 42; or
(7) the VH has a sequence having at least 80% identity or at most 25 mutations compared with the VH corresponding to any one of groups (1) - (6), and the VL has a sequence having at least 80% identity or at most 20 mutations compared with the VL corresponding to any one of groups (1) - (6).

3. The nucleic acid molecule according to any one of claims 1-2, wherein the antigen-binding region is in the form of an scFv having:
(1) the amino acid sequence set forth in SEQ ID NO: 51, 55, or 56; or
(2) the amino acid sequence set forth in SEQ ID NO: 52; or
(3) the amino acid sequence set forth in SEQ ID NO: 53; or
(4) the amino acid sequence set forth in SEQ ID NO: 54 or 57; or
(5) an amino acid sequence having at least 80% identity or at most 50 mutations compared with the amino acid sequence set forth in any one of SEQ ID NOs: 51-57.

4. The nucleic acid molecule according to any one of claims 1-3, wherein the chimeric antigen receptor comprises a CD8α signal peptide, an antigen-binding region that specifically binds to CD19, a CD8 hinge region, a CD8 transmembrane region, a CD28 costimulatory domain, and CD3ζ; preferably,
the CD8α signal peptide has the amino acid sequence set forth in SEQ ID NO: 33 or has an amino acid sequence having at least 80% identity or at most 10 mutations compared with the amino acid sequence set forth in SEQ ID NO: 33, and/or
the CD8α hinge region has the amino acid sequence set forth in SEQ ID NO: 47 or has an amino acid sequence having at least 80% identity or at most 10 mutations compared with the amino acid sequence set forth in SEQ ID NO: 47, and/or
the CD8 transmembrane region has the amino acid sequence set forth in SEQ ID NO: 48 or has an amino acid sequence having at least 80% identity or at most 10 mutations compared with the amino acid sequence set forth in SEQ ID NO: 48, and/or
the CD3ζ has the amino acid sequence set forth in SEQ ID NO: 49 or has an amino acid sequence having at least 80% identity or at most 10 mutations compared with the amino acid sequence set forth in SEQ ID NO: 49, and/or
the CD28 costimulatory domain has the amino acid sequence set forth in SEQ ID NO: 50 or has an amino acid sequence having at least 80% identity or at most 10 mutations compared with the amino acid sequence set forth in SEQ ID NO: 50.

5. The nucleic acid molecule according to any one of claims 1-4, wherein the chimeric antigen receptor further comprises a cytokine linked by a self-cleaving peptide; optionally, the cytokine is IL15 and/or the self-cleaving peptide is selected from F2A, T2A, P2A, and E2A.

6. The nucleic acid molecule according to any one of claims 1-5, wherein the chimeric antigen receptor has the sequence set forth in any one of SEQ ID NOs: 34-37 or 44-46, or has a sequence having at least 80% identity or at most 120 amino acid mutations compared with any one of SEQ ID NOs: 34-37 or 44-46.

7. The nucleic acid molecule according to any one of claims 1-6, wherein the nucleic acid molecule is DNA or RNA; preferably, the RNA is mRNA.

8. A chimeric antigen receptor targeting CD19 encoded by the nucleic acid molecule according to any one of claims 1-7.

9. A vector comprising the nucleic acid molecule according to any one of claims 1-7.

10. An immune effector cell, wherein the immune effector cell is introduced with the nucleic acid molecule according to any one of claims 1-7 or is introduced with the vector according to claim 9 or expresses the chimeric antigen receptor according to claim 8.

11. The immune effector cell according to claim 10, wherein the immune effector cell is an NK cell, a T cell, or an NKT cell, preferably an NK cell.

12. The immune effector cell according to claim 11, wherein the NK cell is differentiated from an iPSC, or derived from peripheral blood or umbilical cord blood.

13. A method for preparing the immune effector cell according to any one of claims 10-12, wherein the method comprises the steps of: providing an immune effector cell, and introducing the nucleic acid molecule according to any one of claims 1-7 or the vector according to claim 9 into the immune effector cell; optionally, the method further comprises culturing the immune effector cell after introducing the nucleic acid molecule or the vector.

14. A product prepared according to the method according to claim 13.

15. A pharmaceutical composition comprising the nucleic acid molecule according to any one of claims 1-7, the vector according to claim 9, the immune effector cell according to any one of claims 10-12, or the product according to claim 14, and a pharmaceutically acceptable carrier.

16. The pharmaceutical composition according to claim 15 for use in treating a tumor or cancer, wherein the tumor or cancer is preferably a CD19-related tumor or cancer, more preferably a B cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

17. Use of the nucleic acid molecule according to any one of claims 1-7, the vector according to claim 9, the immune effector cell according to any one of claims 10-12, the product according to claim 14, or the pharmaceutical composition according to claim 15 in the manufacture of a medicament for treating a tumor or cancer, wherein the tumor or cancer is preferably a CD19-related tumor or cancer, more preferably a B cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.

18. A method for treating a cancer or tumor, wherein the method comprises administering to a subject in need an effective amount of the nucleic acid molecule according to any one of claims 1-7, the vector according to claim 9, the immune effector cell according to any one of claims 10-12, the product according to claim 14, or the pharmaceutical composition according to claim 15, and the tumor or cancer is preferably a CD19-related tumor or cancer, more preferably a B cell malignancy, such as acute myelogenous leukemia, myelodysplastic syndrome, chronic myelogenous leukemia, chronic lymphocytic leukemia, non-Hodgkin's lymphoma, multiple myeloma, plasmacytoma, monoclonal gammopathy of unknown significance, Waldenstrom's macroglobulinemia (lymphoplasmacytic lymphoma), heavy chain disease, primary amyloidosis, post-transplant lymphoproliferative disorder, Hodgkin's lymphoma, MALT lymphoma, B-cell lymphoma, mantle cell lymphoma, (germinal center-like) diffuse large cell lymphoma, Burkitt lymphoma, dual-lineage leukemia, dual-phenotype leukemia, hairy cell leukemia, precursor B acute lymphoblastic leukemia/lymphoma, primary cutaneous follicular center lymphoma, follicular lymphoma, or marginal zone B-cell non-Hodgkin's lymphoma.
